# EUROPEAN PATENT APPLICATION

(11) **EP 1 430 888 A1**
(43) Date of publication of application: **23.06.2004**
(21) Application number: 02772955.7
(22) Date of filing: 27.09.2002
(51) Int. Cl.: A61K 9/24, A61K 47/10, A61K 47/26, A61K 47/36

(54) **PRESS-COATED MOLDED ARTICLE UNDERGOING QUICK DISINTEGRATION**

(30) Priority: 28.09.2001 JP 2001302619
(71) Applicant: Sanwa Kagaku Kenkyusho Co., Ltd., Nagoya-shi, Aichi 461-8631 (JP)
(72) Inventor: HIBINO, T. c/o SANWA KAGAKU KENKYUSHO CO., LTD., Nagoya-shi, Aichi 461-8631 (JP); OZEKI, Yuichi c/o SANWA KAGAKU KENKYUSHO CO., LTD, Nagoya-shi, Aichi 461-8631 (JP); KONDO, Yoshiya c/o SANWA KAGAKU KENKYUSHO CO., LTD, Nagoya-shi, Aichi 461-8631 (JP); WATANABE, Y. c/o SANWA KAGAKU KENKYUSHO CO., LTD., Nagoya-shi, Aichi 461-8631 (JP)
(74) Representative: Torggler, Paul N.
(86) International application number: PCT/JP2002/010131
(87) International publication number: WO 2003/028706

(57) **Abstract**

A press-coated fast-dissolving/disintegrating molded product of the present invention is characterized in that the molded product comprises an inner core that contains ingredients rich in dissolubility or/and disintegrability and whose disintegration time is 1 minute or less and an outer layer that contains ingredients rich in moldability on the outside of the inner core and whose disintegration time is 1 minute or less and that the disintegration time of the entire molded product is 1 minute or less. Preferably, here, ingredients rich in dissolubility or/and disintegrability are further contained in the outer layer and dissolution/disintegration accelerants in the inner core. Thus, by allowing the molded product to have a double structure consisting of an outer layer and inner core and imparting moldability to the outer layer alone - the layer that requires strength - and excellent dissolubility/disintegrability to the inner core, the molded product has been perfected that offers an extremely fast dissolution/disintegration time with sufficient moldability. The present molded product can be readily manufactured by compression molding means having punches above and below a die, with at least the upper punch having a double structure consisting of a center punch and an outer punch enclosing the outer perimeter of the center punch and both the center and outer punches being slidable and manipulatable for compression operation.

## Description

### TECHNICAL FIELD

The present invention relates to a press-coated fast-dissolving/disintegrating molded product manufactured by a set of punches and die through a series of steps and a manufacturing method thereof. More particularly, the present invention relates to a press-coated orally fast-dissolving/disintegrating molded product used in the area of drugs and foods.

### BACKGROUND ART

Recent years have seen efforts led by pharmaceuticals manufacturers to develop tablets that rapidly disintegrate or dissolve in the oral cavity as dosage form that can be readily taken by the elderly having difficulty taking oral pharmaceutical preparations (e.g., tablets, capsules, powders), small children, patients having difficulty taking a tablet due to surgical or medical disease and so on. Further, development of orally fast-dissolving/disintegrating tablets has been demanded from the viewpoint of patients' QOL (Quality of Life) as dosage form that can be taken any time without water. More specifically, normal tablets and capsules present problems such as difficulty in consumption and getting stuck in the pharynx or gullet if taken by the elderly with poor swallowing capability and by small children. As for powders and granules, the drug remains in the oral cavity, making it difficult to swallow and causing unpleasant feelings in the oral cavity. They may result in choking or get stuck between false teeth, producing an unpleasant feelings if taken by the elderly. Further, such oral preparations require water when taken, leading to nighttime urination problem in the case of the elderly and small children. Additionally, it is possible in the case of draft of medicine that they may be consumed under emergency condition, often making it difficult to prepare water. To improve such problems, orally fast-dissolving/disintegrating tablet is an extremely useful dosage form.

Incidentally, while orally fast-dissolving/disintegrating tablet refers to that which is rapidly disintegratable and dissoluble in the oral cavity and offers sufficient moldability (tablet strength) for handling, a manufacturing method of general tablets and their basic physical properties will be briefly described first. Common tablets are generally manufactured by one of the following three methods; the direct tabletting method in which a bulk material that is a mixture of a plurality of ingredients is compression-molded (tabletted) as is, the dry granule compression method in which a bulk material that is a mixture of a plurality of ingredients is granulated and tabletted in a dry form as is and the wet granule compression method in which a bulk material that is a mixture of a plurality of ingredients is granulated after being dampened with an appropriate solvent and then tabletted after being dried. On the other hand, disintegrability, dissolubility and moldability are among the basic physical properties of tablet. Tablet's disintegrability/dissolubility is generally known to be closely related to its moldability. As compression pressure rises, porosity of the bulk material in the tablet declines, providing increased moldability but resulting in reduced disintegrability/dissolubility of the tablet. Conversely, as compression pressure drops, porosity of the bulk material in the tablet increases, leading to reduced moldability but providing higher disintegrability/dissolubility of the tablet. In general, tablet's disintegrability/dissolubility is a parameter opposing to moldability, and in the case of tablets of the same composition, these parameters are determined by compression pressure, that is, porosity of the bulk material in the tablet (Powder Compression Molding Technology edited by Medicine Manufacturing and Particle Design Group of The Society of Powder Technology P.314 to P.315).

Among tablets, orally fast-dissolving/disintegrating tablet is special in that its disintegrability/dissolubility is accelerated (normal disintegration time in the oral cavity is 1 minute or less), with the disintegrability/dissolubility accelerated by enhancing the porosity as compared with normal tablets. As described earlier, however, as the porosity of the tablet increases, moldability - an opposing parameter of disintegrability/dissolubility - drops. For this reason, various manufacturing improvements are made to orally fast-dissolving/disintegrating tablet to maintain necessary moldability for handling while at the same time securing rapid disintegrability/dissolubility by enhancing the porosity of the bulk material in.the tablet. Such methods can be broadly classified into 1. use of techniques not usually employed for tablet manufacturing, 2. addition of manufacturing steps and 3. limitation of ingredient (formulation).

Among examples of "1. use of techniques not usually employed for tablet manufacturing" is a method in which a liquid, in which a medicine, water-soluble gel and saccharides (including sugar alcohols) are dispersed or dissolved, is charged into a pocket of a pre-molded PTP sheet, etc. and then is sealed after freeze-drying to produce a tablet, that is, a method of manufacturing an orally fast-dissolving/disintegrating tablet by freeze-drying step (the method: Japanese Examined Patent Application Publication No. 1-501704, Japanese Unexamined Patent Application Publication No. 3-86837) . Although offering excellent disintegrability/dissolubility, the tablet manufactured by the freeze-drying step has the disadvantage of being extremely low in moldability and brittle. Therefore, the disadvantage, attributed to extremely low moldability and brittleness, is overcome by charging in advance a liquid, into which medicines, etc. are dispersed or dissolved, into a package and molding by freeze-drying. Nevertheless, the disadvantage of difficulty in handling by patients and others attributed to its brittleness has yet to be overcome. Further, conducting freeze-drying, not employed for ordinary tablet manufacturing methods, requires more manufacturing time than with ordinary tablets, resulting in lower productivity and higher cost.

There is another example of the present method in which a wet bulk material containing a medicine and saccharides (including sugar alcohols) is molded or tabletted at a low tabletting pressure (160kg/cm² or less) in a die using a special tabletting machine and then dried to produce a tablet (the method: Japanese Unexamined Patent Application Publication No. 5-271054, Japanese Unexamined Patent Application Publication No. 6-218028). Moldability is enhanced while at the same time providing improved disintegrability and dissolubility by adopting such a method. However, although providing necessary moldability for handling, the tablet manufactured by the wet tabletting compares unfavorably with the freeze-drying method in terms of disintegrability/dissolubility in the oral cavity (oral disintegration time: 18 seconds). Further, the method, besides requiring a special technique for tabletting a wet bulk material (wet tabletting by a special tabletting machine) , involves a troublesome step - drying after wet tabletting. The drying step requires a dryer specifically designed for tablets and uses a belt-type dryer if the orally fast-dissolving/disintegrating tablet lacks moldability and a laminated-container-type ventilating dryer if the orally fast-dissolving/disintegrating tablet has moldability. Moreover, unlike dry bulk material, wet bulk material has absolutely no fluidity, offering extremely high adhesion to metals and so on. Therefore, even if a special tabletting machine designed for wet tabletting is used, an automatic wet bulk material feeding device is required as auxiliary equipment that transfers wet bulk material from the dedicated drum to the tabletting machine's hopper. Additionally, to prevent wet bulk material from adhering to the punches or the die during tabletting, tabletting must be performed via high molecular film, which is a troublesome procedure. (Journal of the Japan Society of Pharmaceutical Machinery and Engineering Vol. 10 No.4 (2001) edited by the Japan Society of Pharmaceutical Machinery and Engineering, P.5 to P.17)

Next, among examples of "2. addition of manufacturing steps to normal tablet manufacturing method" is a method of rendering a tablet porous and fast-soluble by tabletting medicine and saccharides (including sugar alcohols) together with a low-melting point substance at a low tabletting pressure (250kg/cm² or less) followed by heating to melt the low-melting point substance (the method: Japanese Unexamined Patent Application Publication No. 11-35451). Moldability is enhanced while at the same time providing improved disintegrability/dissolubility by adopting such a method. However, although providing necessary moldability for handling, the present method of rendering the tablet porous and fast-soluble by melting the low-melting point substance compares unfavorably with the freeze-drying method in terms of disintegrability/dissolubility in the oral cavity (oral disintegration time: 15 seconds). Further, the post-tabletting heating step (70°C, 60 minutes) for melting the low-melting point substance is not only troublesome but also requires a dryer specifically designed for tablets as with the wet tabletting method described earlier. Besides, it is extremely difficult to uniformly apply a specific temperature to a large number of tablets, thus raising concerns over possible variation in disintegrability/dissolubility between products.

There is another example of the present method in which a tablet is rendered fast-soluble by tabletting a substance, whose composition includes a medicine and amorphous saccharides, at a low tabletting pressure (500kg/cm² or less) and humidifying the surface layer of the obtained tablet (the method: Japanese Unexamined Patent Application Publication No. 11-12162). Moldability is enhanced while at the same time providing improved disintegrability/dissolubility by adopting such a method. However, although providing necessary moldability for handling, the present method of rendering the tablet fast-soluble by humidifying the surface layer thereof compares unfavorably with the freeze-drying method in terms of disintegrability/dissolubility (oral disintegration time: approximately 10 seconds). Further, the method requires a troublesome step of rendering saccharides amorphous by spray drying before tabletting. Additionally, it is not only troublesome to conduct the surface treatment step (e.g., 40°C, 70%, 30 minutes or 25°C, 50%, 120 minutes) but also extremely difficult to uniformly apply a specific temperature to a large number of tablets, thus raising concerns over possible variation in disintegrability/dissolubility between products.

Among examples of "3. limitation of ingredient (formulation) used" is a method of molding a combination of low substituted hydroxypropylcellulose (hereinafter referred to as L-HPC) and saccharides (including sugar alcohols) to produce a tablet (the method: Japanese Unexamined Patent Application Publication No. 11-43429, Japanese Unexamined Patent Application Publication No. 2000-103731). Moldability is enhanced while at the same time providing improved disintegrability/dissolubility by adjusting the amount of L-HPC blended. Although providing necessary moldability for handling, the present method of molding a combination of L-HPC and saccharides (including sugar alcohols) to produce a tablet compares unfavorably with the freeze-drying method in terms of disintegrability/dissolubility (oral disintegration time: 17 seconds) . Further, addition of L-HPC and saccharides (including sugar alcohols) is inevitably necessary. Moreover, the hydroxypropoxyl group content in the L-HPC used must be 7.0 to 9.9 % (w/w) (standard content of hydroxypropoxyl group in L-HPC is 5.0 to 16.0 %(w/w)) - an extremely limited ingredient, which implies that the method lacks versatility.

Thus, conventional orally fast-dissolving/disintegrating tablets showed declines in moldability with increasing disintegrability/dissolubility whereas their disintegrability/dissolubility dropped with increasing moldability. In other words, while conventional orally fast-dissolving/disintegrating tablets excelled in one of the two opposing physical properties, they.were not satisfactory in the other property. Further, it was essential to use saccharides (including sugar alcohols) having excellent dissolubility and sufficient moldability as basic ingredients.

### DISCLOSURE OF THE INVENTION

In light of the above, the present inventors have perfected the present invention with an object of providing a fast-dissolving/disintegrating molded product that rapidly disintegrates or/and dissolves in the oral cavity while at the same time providing sufficient moldability. The present invention further provides a fast-dissolving/disintegrating molded product manufacturing technique that eliminates the needs to use any costly technique different from common tablet manufacturing methods and troublesome techniques such as drying step after tabletting and heating step after tabletting and further eliminates the need to limit ingredient (formulation).

The present inventors have arrived at a conclusion that the fact that conventional fast-dissolving/disintegrating tablets are made up of the same composition from the outside to the inside makes it difficult to satisfy the two opposing properties required of orally fast-dissolving/disintegrating tablets, namely, disintegrability/dissolubility on the one hand and moldability (tablet strength) on the other hand. For this reason, the inventors have perfected the present invention by devising an idea of using a double structure consisting of an inner core and an outer layer for a molded product to be molded. Adopting such a structure has made it possible to impart moldability to the outer layer alone that requires strength and outstanding dissolubility or/and disintegrability to the inner core. Moreover, double structure allows for use of different ingredients for the inner core and the outer layer, thus making it possible to readily manufacture a press-coated fast-dissolving/disintegrating molded product that has excellent dissolubility or/and disintegrability and also provides sufficient moldability.

The press-coated fast-dissolving/disintegrating molded product of the present invention is characterized in that it has an inner core containing ingredients rich in dissolubility or/and disintegrability and whose disintegration time is 1 minute or less and an outer layer containing ingredients rich in moldability provided outside the inner core and whose disintegration time is 1 minute or less and that the disintegration time of the molded product as a whole is 1 minute or less. Here, it is preferred that the outer layer further contain an ingredient rich in dissolubility or/and disintegrability. It is also preferred that the inner core contain a dissolution/disintegration accelerant. It is also preferred that the molded product of the present invention be constituted so as to offer a friability of 5% or less (25 rpm, 4 minutes) . It is to be noted that crystalline cellulose is preferably among the ingredients rich in moldability whereas mannitol, erythrytol and lactose are preferably among the saccharides and sugar alcohols.

In the present invention, a method of molding a molded product having a core in a single step, which is an integral molding method, and an apparatus therefor have been devised, thus allowing molding of a tablet having a double structure consisting of an inner core and an outer layer at high productivity and with ease. That is, the molded product of the present invention can be readily manufactured by compression molding means having punches above and below a die, with at least the upper punch having a double structure consisting of a center punch and an outer punch enclosing the outer perimeter of the center punch and both the center and outer punches being slidable and manipulatable for compression operation. Thus, the press-coated fast-dissolving/disintegrating molded product of the present invention can be manufactured in a single step by a single tabletting machine, eliminating the needs for conventional troublesome techniques/steps and ensuring efficiency in manufacture.

### BRIEF DESCRIPTION OF THE DRAWINGS

Figs. 1 illustrate explanatory views of punch tip operations showing a first example of a manufacturing method of a press-coated fast-dissolving/disintegrating molded product of the present invention (shading as cross section omitted);
Figs. 2 illustrate explanatory views of punch tip operations showing a second example of the manufacturing method of the press-coated fast-dissolving/disintegrating molded product of the present invention (shading as cross section omitted);
Figs. 3 illustrate explanatory views of punch tip operations showing a third example of the manufacturing method of the press-coated fast-dissolving/disintegrating molded product of the present invention (shading as cross section omitted);
Figs. 4 illustrate explanatory views of punch tip operations showing a partial modification of the third example of the manufacturing method of the press-coated fast-dissolving/disintegrating molded product of the present invention (shading as cross section omitted);
In Figs. 5 that illustrate an example of a double-structured punch used in the present invention showing an upper punch, Fig. 5 (A) illustrates a vertical sectional view (right half) and a schematic view (left half), and Fig. 5 (B) a side view, with the double punch corresponding to Fig. 9;
Fig. 6 illustrates an example of a double-structured punch used in the present invention showing an upper punch - a vertical sectional view (right half) and a schematic view (left half) - with the double punch corresponding to Fig. 10;
Fig. 7 illustrates an overall front sectional view of a common rotary compression molding machine, except that the sectional views of the punch, the vertical shaft, and the hopper are not shown;
Fig. 8 illustrates a schematic plan view showing the top side of a turntable in an embodiment of the rotary compression molding machine of the present invention;
Fig. 9 illustrates a schematic view including a partial sectional view and showing the operational mechanism of upper and lower punches by developing the turntable in an embodiment of the rotary compression molding machine of the present invention;
Fig. 10 illustrates a schematic view including a partial sectional view and showing the operational mechanism of upper and lower punches by developing the turntable in an embodiment of the rotary compression molding machine of the present invention, which is the reverse of the operation system of the outer and center punches in Fig. 9;
In Figs. 11 that illustrate a residual bulk material removal device of the present invention, Fig. 11(A) illustrates a bird's-eye view, and Fig. 11 (B) a top view; and
In Figs. 12 that illustrate an embodiment of a molded product manufactured by the manufacturing method and apparatus of the press-coated fast-dissolving/disintegrating molded product of the present invention, Fig. 12(A) a side view, Fig. 12 (B) a top view, and Fig. 12 (C) a longitudinal sectional perspective view.

### BEST MODE FOR CARRYING OUT THE INVENTION

The press-coated fast-dissolving/disintegrating molded product of the present invention refers to a molded product that rapidly dissolves or/and disintegrates, and more specifically refers to a molded product whose disintegration time is 1 minute or less when a disintegration test is conducted in accordance with the Disintegration Test of the General Tests, Processes and Apparatus of the Japanese Pharmacopoeia, Fourteenth Edition. The disintegration time is preferably 30 seconds or less and more preferably 10 seconds or less. When the molded product is used as an orally fast-dissolving/disintegrating molded product, it has been confirmed that the actual dissolution/disintegration time in the oral cavity is equivalent to or shorter than the disintegration time obtained from the aforementioned Disintegration Test as a result of addition of friction within the oral cavity and tongue movement.

It is to be noted that the aforementioned Disintegration Test can be briefly summed up as follows. The basket-rack assembly is attached to the bearing, immersed in the fluid in a beaker, and adjusted the apparatus so as to raise and lower the basket smoothly at a constant frequency of 29 to 32 cycles per minute through a distance of 53 to 57 mm. At the lowest point of the downward stroke, the wire mesh must be 25mm distance from the bottom of the beaker and the volume of the fluid in the beaker is such that, at the lowest point of the downward stroke, the top of the basket is on a level withy the surface of the fluid. The temperature of the fluid is maintained at 37±2°C during the test. The test is carried out using water as the test fluid. Observe the tablets after 30 minutes of operation with auxiliary disks: the tablets comply with the test, if no residue remains in the glass tube.

Ingredient rich in dissolubility or/and disintegrability in the present specification refers to a single ingredient or a mixture of a plurality of ingredients (including granulated substance) blended with a dissolution/disintegration accelerant and others whose disintegration time is 1 minute or less, when 160mg of the single ingredient or the mixture of the plurality of ingredients molded using punches and die of 8mm in diameter at a tabletting pressure of 100kg/tablet is subjected to a disintegration test in accordance with the Disintegration Test.

While moldability denotes molded product strength and may be expressed in terms of friability, hardness, etc., a molded product is defined as having high moldability in the present specification when its friability is 5% or less especially at 25 rpm for four minutes, with the cumulative number of revolutions set to 100, in a frialibility test described later. Conversely, a molded product is defined as having low moldability if its degree of friability is 5% or more, and such a molded product with low moldability is referred to as imperfect molded product. In the present specification, ingredient rich in moldability refers to a single ingredient or a mixture of a plurality of ingredients (including granulated substance) blended with a filler,etc. whose degree of friability is 0.5% or less, when 160mg of the single ingredient or the mixture of the plurality of ingredients molded using punches and die of 8mm in diameter at a tabletting pressure of 300kg/tablet at 25 rpm for 4 minutes is subjected to the friability test described later.

Here, the term "friableness" conveys a meaning used normally in the technical field of pharmaceutical preparations and, namely, it is a parameter evaluated in terms of decline in tablet weight in an friablity tester using a rotary drum in order to determine whether a tablet is capable of withstanding succeeding steps such as coating, printing and packaging as well as vibrations and shocks that may arise in the market and distribution. More specifically, tablet weight is measured after a given number of revolutions, with a motor-equipped drum (inner diameter: 287±4mm) adjusted to, for example, 24 to 26 revolutions per minute in accordance with reference information "Tablet Friability Test" in the Japanese Pharmacopoeia, Fourteenth Edition. Friability is determined by calculating the percentage of decline in weight relative to the tablet weight at the beginning of the test.

While hardness, on the other hand, is an indicator for evaluating how hard a tablet is as described earlier, it is not appropriate to evaluate moldability of the entire tablet by the compression destructive test commonly used in the fields of drugs and foods if the tablet has a double structure as in the case of the present invention. In the present description, for this reason, moldability is assessed primarily by friability test while hardness evaluation is confined to confirming that the tablet is hard enough not to cause inconvenience for handling.

Further, in the present specification, an active ingredient (effective ingredient, main ingredient) in drugs, a main ingredient in foods and an ingredient for manifesting the main function in other molded products are worded as a "main ingredient" whereas ingredients other than the main ingredient, namely, various additives such as filler, binder, disintegrator, lubricant and anti-agglutinator that are regularly used in the field of formulation technology are worded collectively and in a broad sense as "filler, etc."

The press-coated fast-dissolving/disintegrating molded product of the present invention contains ingredients rich in moldability in the outer layer and preferably further contains those rich in dissolubility or/and disintegrability. On the other hand, the press-coated fast-dissolving/disintegrating molded product of the present invention contains ingredients rich in dissolubility or/and disintegrability in the inner core and further contains a dissolution/disintegration accelerant under certain circumstances. Such ingredients used in the inner core and the outer layer satisfy the aforementioned target disintegration time, that is, these ingredients ensure that the respective disintegration times of the inner core and the outer layer and that of the molded product as a whole are 1 minute or less, preferably 30 seconds or less and further preferably 10 seconds or less. It is also preferred that the friability (25 rpm, 4 min.) of the molded product of the present invention be 5% or less, more preferably 1% or less and further preferably 0.5% or less. That is, it is preferred that ingredients, used in the inner core and the outer layer, satisfy the criteria for the friability.

The press-coated fast-dissolving/disintegrating molded product of the present invention is basically characterized in that it has a double structure containing ingredients rich in dissolubility or/and disintegrability in the inner core and ingredients rich in moldability in the outer layer. Consequently, ingredients rich in moldability contained in the outer layer provide moldability, thus allowing for large quantities of ingredients generally poor in moldability but rich in dissolubility or/and disintegrability to be contained in the inner core. In particular, since the outer layer must satisfy the aforementioned criteria for disintegration time while maintaining its moldability, ingredients rich in dissolubility or/and disintegrability are required if the outer layer is thick, and the quantities required are propartal to the outer layer thickness. As for the inner core, it may be possible to provide further improved dissolubility or/and disintegrability by adding a dissolution/disintegration accelerant. It is to be noted that there are two cases for using a dissolution/disintegration accelerant; one in which a dissolution/disintegration accelerant is added further to ingredients rich in dissolubility or/and disintegrability and the other in which ingredients lacking in dissolubility or/and disintegrability are transformed into those rich therein by addition of a dissolution/disintegration accelerant.

Among ingredients rich in moldability to be contained in the outer layer are sorbitol, crystalline cellulose, hydroxypropylcellulose and povidone. Sorbitol and crystalline cellulose are preferred above all others, and crystalline cellulose is further preferred.

Among ingredients rich in dissolubility or/and disintegrability to be contained in the inner core and, under certain circumstances, in the outer layer are commonly saccharides and sugar alcohols that particularly hold promise of fast disintegrability in the oral cavity and are widely used as filler, etc. in the fields of drugs and foods, that is, such ingredients include glucose, xylose, lactose, sucrose, maltose, xylitol, mannitol, multitol, erythritol and lactitol. Ingredients preferred for the outer layer are lactose, mannitol and erythritol. Ingredients preferred for the inner core are xylose, lactose, maltose, xylitol, mannitol and erythritol, and xylose, xylitol and erythritol are further preferred.

Among dissolution/disintegration accelerants to be contained in the inner core are carmellose, carmellose calcium, carmellose sodium, crosscarmellose sodium, low substituted hydroxypropylcellulose, carboxymethyl starch sodium, corn starch, potato starch, alpha starch, partial alpha starch, hydroxypropyl starch, crosspovidone, sodium laurylsulfate, polysorbate, polyoxypropylene-polyoxyethylene glycol, sorbitan monooleate, propylene glycol monostearate and polyethylene glycol monolaurate. Carmellose calcium, carboxymethyl starch sodium, corn starch, hydroxypropyl starch and crosspovidone are preferred above all others, and corn starch is further preferred.

Such ingredients to be contained in the outer layer and the inner core are not specifically limited as long as they are usable as common filler, etc. It is also possible to select a plurality of ingredients from among those listed above and contain them. It is to be noted that while the ingredients rich in moldability, those rich in dissolubility or/and disintegrability and dissolution/disintegration accelerants are usually ingredients other than main ingredients and used as filler, etc., it is also possible to adopt a special embodiment in which such ingredients are contained as main ingredients.

When an ingredient rich in dissolubility or/and disintegrability is further blended with a dissolution/disintegration accelerant in the inner core, it is preferred that the blending ratio be 19:1 to 3:7, for example, for the inner core of 6mm in diameter at a tabletting pressure of 500 to 2000 kg/tablet, and it is further preferred that the blending ratio be 19:1 to 7:3. It is to be noted that the present blending ratio suited for fast-dissolving/disintegrating molded products is not limited to the range as it is affected by the molded product size and the tabletting pressure.

When an ingredient rich in moldability is further blended with an ingredient rich in dissolubility or/and disintegrability in the outer layer, it is preferred that the blending ratio be 7:1 to 2:6, for example, for the inner core of 6mm in diameter and the outer layer of 8mm in diameter at a tabletting pressure of 500 to 2000 kg/tablet, and it is further preferred that the blending ratio be 6:2 to 4:4. It is to be noted that the present blending ratio suited for fast-dissolving/disintegrating molded products is not limited to the range as it is affected by the molded product size, the tabletting pressure and the outer layer thickness. When the outer layer is thick, the percentage of the ingredient rich in dissolubility or/and disintegrability to be blended is enhanced whereas when the outer layer is thin, the percentage of the ingredient rich in moldability to be blended is enhanced. If the outer layer is extremely thin, ingredient rich in dissolubility or/and disintegrability may be unnecessary.

As explained above, among specific examples of blending of ingredients in the inner core and the outer layer are a combination of crystalline cellulose and mannitol, that of crystalline cellulose and erythritol and that of crystalline cellulose and lactose for the outer layer, for example, when a combination of erythritol and mannitol is used for the inner core. Here, it is preferred that the erythritol particle diameter be 75 µm (200 mesh), for example, for the inner core of 6mm in diameter and the outer layer of 8mm in diameter at a tabletting pressure of 500 to 2000 kg/tablet, and it is further preferred that the particle diameter be 150 to 710 µm (22 to 100 mesh).

It is to be noted that an ingredient used for the inner core can be transformed into that rich in dissolubility or/and disintegrability different from its original basic property by mixing an ingredient lacking in dissolubility or/and disintegrability and a dissolution/disintegration accelerant or by performing granulation or other step in a dry or wet form. It is also possible to eventually bring about the substantially same condition as when an ingredient rich in dissolubility or/and disintegrability is contained by first tabletting an ingredient lacking in dissolubility or/and disintegrability (including when a plurality of ingredients are blended) and then heating or/and humidifying the molded product for reforming only the surface or down through the inside of the added ingredient, and such cases are also construed as being encompassed within the technical scope of the present invention.

It is to be noted that an ingredient used for the outer layer can be transformed into that rich in moldability different from its original basic property by mixing an ingredient lacking in moldability and a filler, etc. or by performing granulation or other step in a dry or wet form. It is also possible to eventually bring about the substantially same condition as when an ingredient rich in moldability is contained by first tabletting an ingredient lacking in moldability (including when a plurality of ingredients are blended) and then heating or/and humidifying the molded product for reforming only the surface or down through the inside of the added ingredient, and such cases are also construed as being encompassed within the technical scope of the present invention.

Thus, the press-coated fast-dissolving/disintegrating molded product of the present invention has resolved the two opposing phenomena - improved dissolubility or/and disintegrability and improved moldability (as moldability improves, dissolubility or/and disintegrability deteriorate) - by adopting a double structure in the molded product and unevenly distributing ingredients rich in moldability. That is, by unevenly distributing ingredients rich in moldability in the outer layer, it is possible to produce a molded product capable of maintaining its moldability by the outer layer alone whose inner core consists of an imperfect molded substance. Here, the term "imperfect molded substance" refers to a molded product whose friability is 5% or more at 25 rpm for 4 minutes, as defined above. In the case of the press-coated fast-dissolving/disintegrating molded product of the present invention, it is possible to further reduce the inner core moldability and produce a molded product having the inner core whose friability is 5% or more at 25 rpm for four minutes. There are no problems with the outer layer moldability, which is none other than the moldability of the entire molded product as long as the friability is 5% or less at 25 rpm for 4 minutes. It is to be noted that the tolerance level for the outer layer moldability may be more rigorous depending on the application of the molded product.

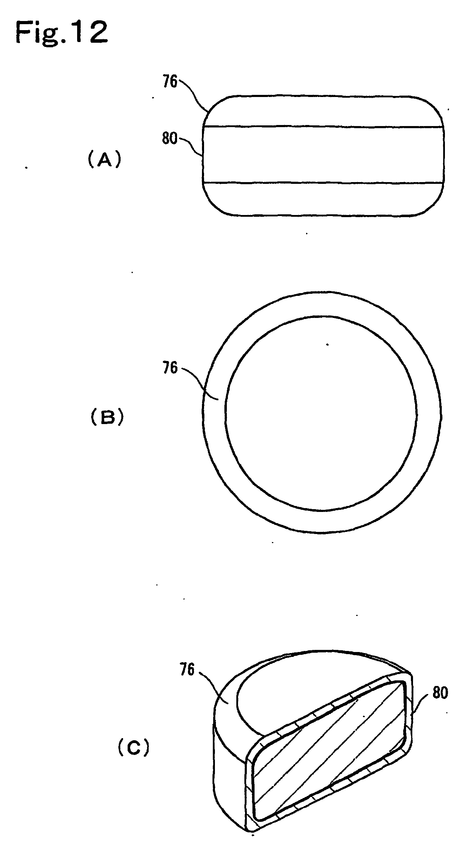

The press-coated fast-dissolving/disintegrating molded product of the present invention is manufactured by integral molding, keeping the core displacement-free as is found in conventional press-coated molded products and thereby making it possible to achieve an extremely thin outer layer. In particular, the press-coated fast-dissolving/disintegrating molded product of the present invention has allowed for the outer perimeter layer thickness entirely to be reduced to 1mm or less, 0.9mm or less and further 0.5mm or less. The present reduction in outer layer thickness also contributes to improved dissolubility or/and disintegrability of the molded product. Here, the term "outer perimeter layer", that is a part determined by the gap between the center punch and the die, is defined as the outer layer part on the side surface part of a molded product that consists of surfaces perpendicular to the diameter surfaces of the molded product (surfaces perpendicular to the direction in which pressure is applied) (80 in Fig. 12) . Since the thicknesses of the upper and lower parts of the molded product's outer layer are adjustable by changing in the amount of bulk material supplied even in the conventional press-coated tablet manufacturing technology, the superiority of the present invention is demonstrated by consciously rewording the molded product thickness as outer perimeter layer here. Here, the term "integral molding" refers to compression molding through a series of steps by using only a set of punches and die. The term has a meaning in contrast with conventional press-coated molded products manufactured by molding a core in advance and supplying it in the middle of molding process.

While applicable to a variety of fields, the press-coated fast-dissolving/disintegrating molded product of the present invention is particularly best suited to orally ingested molded product or fast-dissolving/disintegrating molded product in the oral cavity.

Applications of the press-coated fast-dissolving/disintegrating molded product of the present invention to drugs will be described further in detail below.

It suffices for the press-coated fast-dissolving/disintegrating molded product of the present invention to be shaped so as to be easy to hold or so as not to cause malaise when consumed, and the shape thereof is not specifically limited. However, it is preferred that the molded product be round or elliptical as with ordinary drugs. It suffices for the molded product of the present invention to be sized so as to be insertable into the oral cavity and so as not to involve difficulties in deglutition. For instance, it suffices, in the case of a circular tablet, to design the tablet to be about 25mm or less in diameter or 4 to 25mm in diameter, and preferably 6 to 16mm in diameter and more preferably 8 to 12mm in diameter.

The shape of the inner core, while dependent on the shape of the punch tip described later in the manufacturing method, conforms to the shape of the press-coated fast-dissolving/disintegrating molded product. It is not preferred that the inner core, often dependent on the size of the entire molded product, be excessively small for smooth molding step of the inner core. It is preferred that the inner core be sized large relative to the outer layer for improved dissolubility or/and disintegrability of the molded product to the extent that molding of the outer layer is not hindered. Therefore, it suffices, in the case of a round tablet, to design the inner core to be about 24mm or less in diameter or 3 to 24mm in diameter, and preferably 5 to 15mm in diameter and more preferably 7 to 11mm in diameter. It is to be noted that the inner core may be divided, as described later, into a plurality of parts as necessary.

As for the outer layer thickness, it suffices to design the thickness in accordance with the inner core size so as to have low abrasiveness and be able to maintain the shape of the molded product by the outer layer, and the thickness in the range of 0.2 to 2mm would be appropriate. To enhance fast dissolubility and disintegrability in the oral cavity, it is better not to increase moldability (tablet strength) of the outer layer part more than necessary. For this reason, it is preferred that the outer layer be reduced in thickness as much as possible to the extent that no abrasiveness-related problems arise, that is, to the extent that the shape of the molded product can be maintained, and the thickness of 0.5 to 1mm would be realistic and preferred.

In the press-coated fast-dissolving/disintegrating molded product of the present invention, the main ingredients such as active pharmaceutical ingredients, while normally contained in the inner core, can be contained, as necessary, in the outer layer. In addition to the aforementioned ingredients, a variety of additives regularly used in the field of formulation technology such as filler, binder, disintegrator, lubricant and anti-agglutinator (already collectively defined as "filler, etc.") may be blended in the outer layer and the inner core of the molded product of the present invention, although this partially overlaps the description given earlier. As for the amount of addition thereof, such additives can be used without any problem in amounts based on the knowledge regularly used in the field of formulation technology. It is also possible to obtain a pleasant feeling of consumption by flavoring or scenting the outer layer and/or the inner core by blending sweeteners and scenting agents regularly used in the field of formulation technology.

It is to be understood that while ingredients to be contained in the inner core and the outer layer can be used as is, granulated granular substance may be prepared once by granulation by a normal method and sized as necessary for use. It is also possible to prepare granulated granular substance by coating the main ingredient and a binder on an inactive carrier. In the case of an active ingredient of drugs, granulated granular substance may be further coated as necessary with sustained release coating, time lag coating, enteric coating, gastric coating, water soluble coating, etc. If such coating ingredients are contained, sustained release press-coated orally fast-dissolving/disintegrating molded product or enteric press-coated orally fast-dissolving/disintegrating molded product is produced.

Next, a description will be given below of the manufacturing method of the press-coated molded product of the present invention. In the present description, the term "bulk material" is used to represent all molding materials including powders and granules, except where the term "powder" is particularly commonly used. It is to be noted that the present manufacturing method is described in detail in WO01/98067, although not disclosed at the time of the priority date of the present application, to be disclosed at the time of the convention application.

The press-coated fast-dissolving/disintegrating molded product of the present invention can be manufactured by compression molding means having punches above and below a die, with at least the upper punch having a double structure consisting of a center punch and an outer punch enclosing the outer perimeter of the center punch and both the center and outer punches being slidable and manipulatable for compression operation. Here, a punch, having a double structure consisting of a center punch and an outer punch enclosing the outer perimeter of the center punch, is also normally used for the lower punch, with both the center and outer punches being slidable and manipulatable for compression operation.

The present manufacturing method is a manufacturing method of a press-coated molded product that includes supply steps for bulk materials respectively for the inner core and the outer layer, compression molding steps for the bulk materials for the inner core and/or the outer layer and a compression molding step for the entire molded product containing the inner core. Here, it is preferred that temporary compression be performed in the compression molding steps for the bulk materials for the inner core and/or the outer layer. The supply step for the bulk material for the outer layer is normally carried out twice or more. In this case, depending on the shape of the punch tip, it is further necessary to perform a step for removing residual bulk material remaining on the lower outer punch and/or the molded product, or it may be preferred to do SO.

A preferred embodiment of the manufacturing method of the molded product of the present invention is worded as a "manufacturing method of a press-coated molded product using compression molding means having punches above and below a die, with both the upper and lower punches having a double structure consisting of a center punch and an outer punch enclosing the outer perimeter of the center punch and both the center and outer punches being slidable and manipulatable for compression operation, the manufacturing method including an outer layer supply step 1, in which a bulk material for the outer later is supplied into a space enclosed by a lower outer punch and above a lower center punch, an inner core supply step, in which a bulk material for the inner core is supplied into a space enclosed by the lower outer punch and above the bulk material for the outer layer supplied in the previous step, an outer layer/inner core molding step in which the bulk materials for the outer layer and the inner core supplied by the time of the previous step are compression-molded, an outer layer supply step 2 in which a bulk material for the outer layer is further supplied into a space above and around the molded products of the outer layer and the inner core in the die molded in the previous step and an overall molding step in which the molded products of the outer layer and the inner core and the bulk material for the outer layer are compression-molded." Here, when ordinary bulk materials are used as molding materials, it is preferred that the outer layer molding step be carried out for compression-molding of a bulk material for the outer layer immediately after the outer layer supply step 1 from the viewpoint of preventing contamination between the bulk materials for the outer layer and the inner core and making a clear distinction between outer layer and inner core parts. It is also preferred that temporary compression be carried out as compression operation in the outer layer/inner core molding step and the outer layer molding step as described above. A molded product made in this case can be called temporary molded product, and molded products include temporary molded products. While only main compression may be performed as compression operation in the overall molding step, it is preferred that precompression (temporary compression) be carried out first followed by main compression. Thus, temporary compression is designed to enhance integrality of finally-produced molded products, thus allowing for manufacture of molded products with excellent friability resistance.

The present invention also allows for manufacture of a press-coated fast-dissolving/disintegrating molded product having a plurality of inner cores. The manufacturing method in the aforementioned embodiment makes it possible to readily manufacture a press-coated fast-dissolving/disintegrating molded product having a plurality of inner cores by repeating part of the steps thereof. That is, it is possible to readily manufacture a press-coated fast-dissolving/disintegrating molded product having a plurality of inner cores by further performing, after the inner core supply step for supplying a bulk material for the inner core, a repetitive outer layer/inner core supply step in which the inner core or outer layer supply step is performed once or more for supplying a bulk material for the inner core or the outer layer into a space enclosed by the lower outer punch and above the bulk materials supplied by the time of the previous step. Here, whether to supply a bulk material for the outer layer or the inner core can be selected as necessary, and if a bulk material for the inner core is supplied as the repetitive outer layer/inner core supply step, it is possible to manufacture a press-coated fast-dissolving/disintegrating molded product in which two inner cores exist continuously. If bulk materials for the outer layer and the inner core are supplied in succession as the repetitive outer layer/inner core supply step, it is possible to manufacture a press-coated fast-dissolving/disintegrating molded product in which two inner cores exist, separated by the outer layer. Naturally, it is also possible to separate the two inner cores by supplying, in place of a bulk material for the outer layer, a bulk material for the inner core containing all but the main ingredient. A multi-core press-coated fast-dissolving/disintegrating molded product can be readily manufactured by repeating the present step a number of times. When ordinary bulk materials are used as molding materials, it is also preferred in the repetitive outer layer/inner core supply step that a compression molding step be carried out as described earlier after each supply of a bulk material.

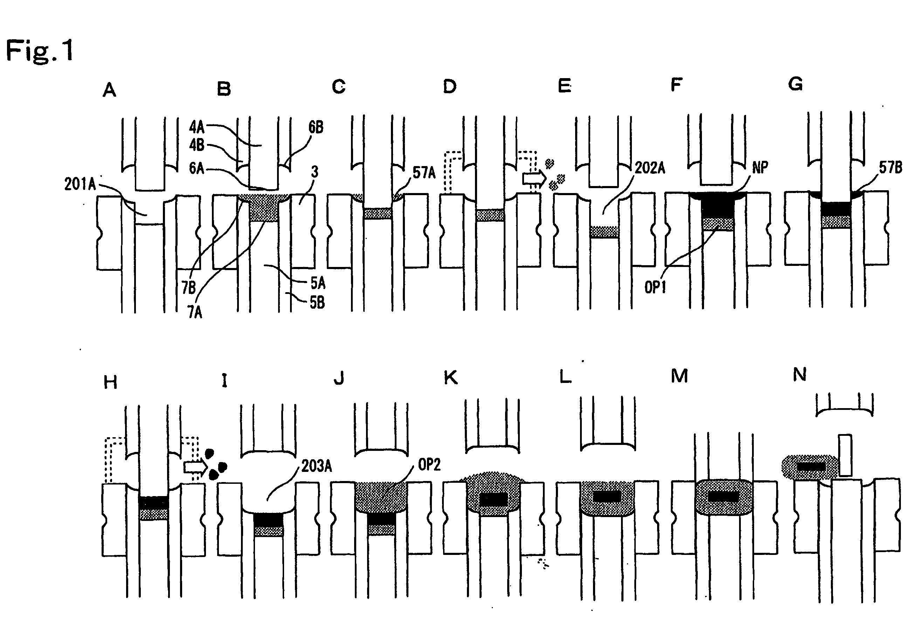

A first example, believed to be the most preferred embodiment of the molded product manufacturing method of the present invention, will be described below in detail mainly with reference to Figs. 1. It is to be noted that temporary compression is used as midway compression operation and that temporary compression operation for a bulk material for a first outer layer OP1 is performed without being omitted. Wordings such as a bulk material for the first outer layer OP1 and a bulk material for a second outer layer OP2 are not used to mean different bulk materials but used for convenience to make a distinction between the parts.

First, with a lower center punch 5A lowered (Fig. 1A), a bulk material for the first outer layer OP1 is supplied into a first outer layer space 201A enclosed by a lower outer punch 5B and above the lower center punch 5A (Fig. 1B). After raising the lower center punch 5A as necessary and thus discharging the excess bulk material for the first outer layer OP1 out of the die, an upper center punch 4A and the lower center punch 5A are moved in mutually approaching directions for temporary compression (Fig. 1C), thus temporarily molding the first outer layer.

Next, with the temporary molded product of the first outer layer OP1 held by the lower center and outer punches 5A and 5B, a bulk material for an inner core NP is supplied into an inner core space 202A enclosed by the lower outer punch 5B and above the temporary molded product of the first outer layer OP1 by lowering the lower center punch 5A as necessary (Figs. 1E and 1F). Then, after raising the lower center punch 5A as necessary and thus discharging the excess bulk material for the inner core out of the die, the upper center punch 4A and the lower center punch 5A are moved in mutually approaching directions for temporary compression (Fig. 1G), thus temporarily molding the temporary molded products of the first outer layer and the inner core.

Further, with the temporary molded products of the first outer layer and the inner core held by the lower center and outer punches 5A and 5B, a bulk material for the second outer layer OP2 is supplied into a second outer layer space 203A above and around the temporary molded products of the first outer layer and the inner core within a die 3 (Figs. 1J and 1K) by lowering the lower punch (both the lower center and outer punches 5A and 5B or the lower outer punch 5B) (Fig. 1I). The temporary molded product of the inner core held on the temporary molded product of the first outer layer is allowed to be completely covered with a bulk material for the outer layer and the temporary molded product of the outer layer (Fig. 1K) , and the excess bulk material for the second outer layer OP2 is discharged as necessary out of the die 3 (Fig. 1L) . It is to be noted that the bulk material for the second outer layer OP2 can be supplied after sufficiently lowering the lower outer punch 5B first such that the temporary molded products of the first outer layer and the inner core are apparently pushed up. Then, the upper punch (both the upper center and outer punches 4A and 4B) and the lower punch (both the lower center and outer punches 4A and 4B) are moved in mutually approaching directions for precompression (temporary compression) of the entire molded product consisting of the first outer layer, the inner core and the second outer layer as necessary, eventually followed by main compression (Fig. 1M). (Overall molding step)

The step shown in Fig. 1N is for ejecting the completed molded product.

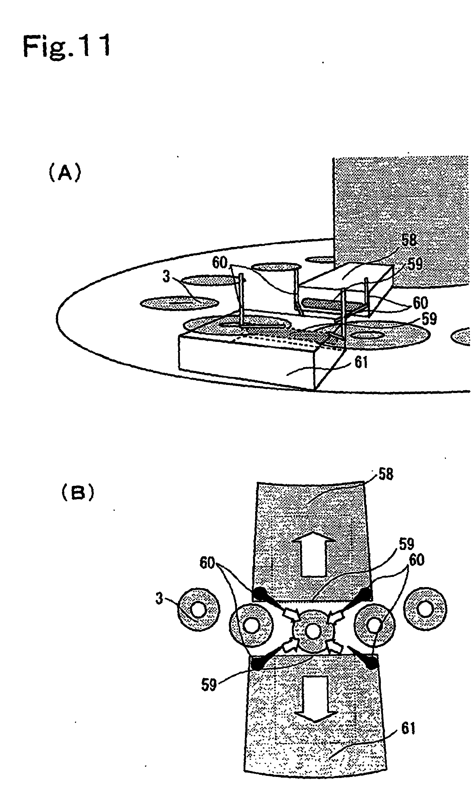

It is to be noted that outer punch tip parts (6B, 7B) correspond to a circumferential edge 76 of a completed molded product shown in Figs. 12 and may be flat depending on the embodiment of the molded product. However, if they are not flat as shown in Figs. 1, it is preferred, to prevent contamination between the bulk materials for the outer layer and the inner core, that steps (Figs. 1D and 1H) for removing residual bulk materials 57 (57A and 57B) remaining on an upper surface 7B of the lower outer punch be further added after supply of the first outer layer OP1 or during compression molding thereof (during temporary molding) or thereafter and after supply of the inner core NP or during compression molding of the first outer layer OP1 and the inner core NP (during temporary molding) or thereafter. The present removal steps can be carried out with compressed air injection and suction (device shown in Figs. 11) or brushing, scraper, etc. or a combination thereof. These are referred to as residual bulk material removal means.

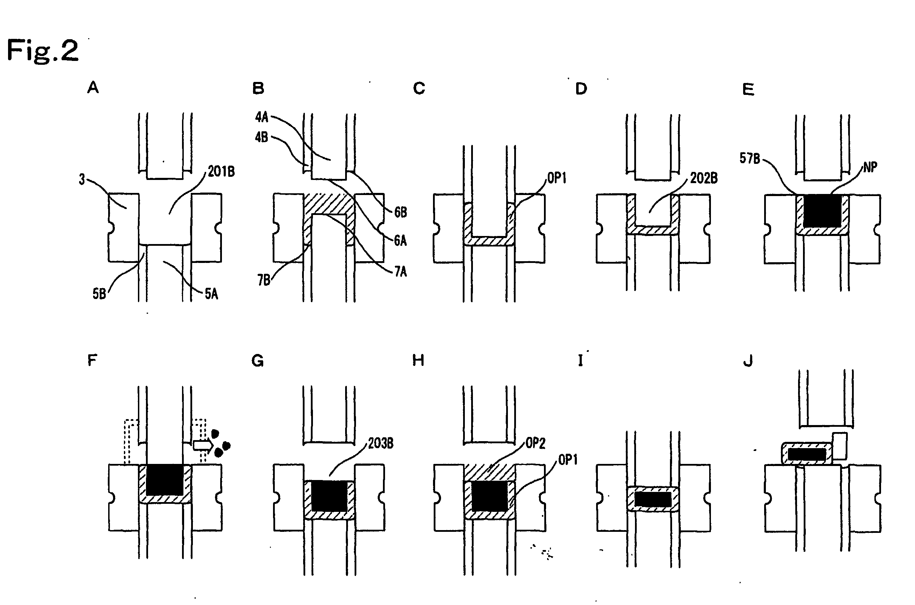

Next, a second example of the molded product manufacturing method of the present invention is a manufacturing method in which a pot-shaped temporary molded product of the first outer layer is made first, a bulk material for the inner core is put in the molded product and a bulk material for the outer layer is supplied again at the end for manufacture of a molded product as shown in Figs. 2. In the present method, it is preferred that a step (Fig. 2F) be added of removing the bulk material 57B for the inner core adhering to the upper part of the temporary molded product of the first outer layer. In the present method, a normal punch with no double structure may be used for the lower punch, and charging of bulk materials, while being problematic to some extent, can be performed without any problem as long as the inner core is small in quantity.

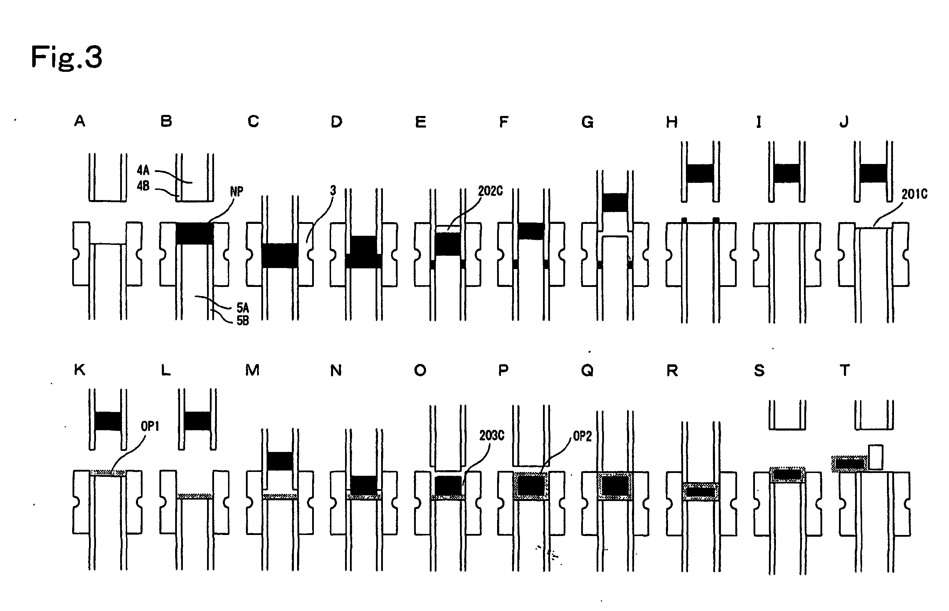

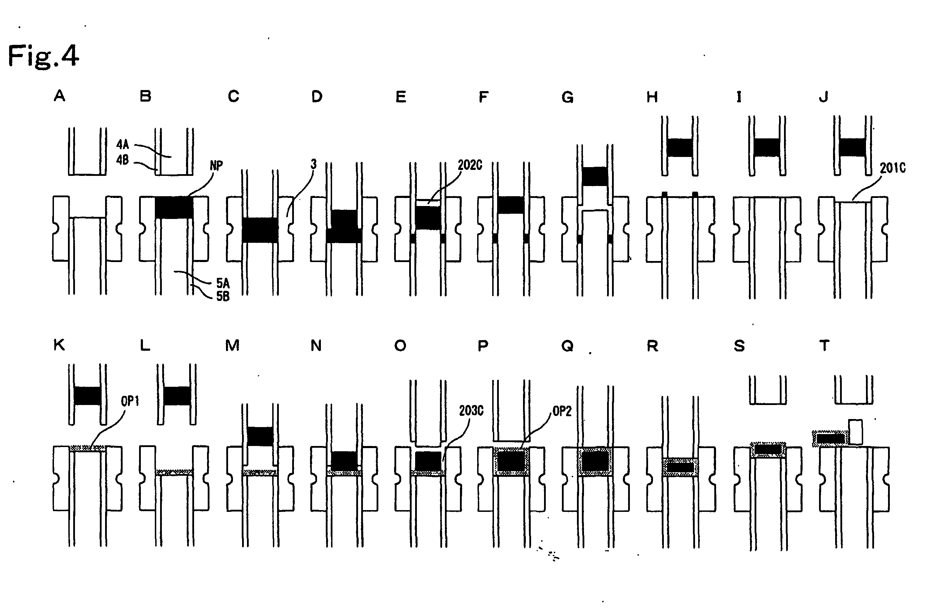

Next, a third example of the molded product manufacturing method of the present invention is a manufacturing method in which the core is molded and lifted in the upper punch first, followed by supply of the first outer layer, lowering of the core back on top of the first outer layer and supply of the second outer layer as shown in Figs. 3. In the present method, a supply method as shown in Figs. 4 may be used for the bulk material for the core.

In the third example of the molded product manufacturing method of the present invention, it is possible to charge the bulk material for the inner core NP into a space above the lower center punch 5A and enclosed by the lower outer punch 5B and lower the upper punch (the upper center and outer punches 4A and 4B), thus moving the bulk material for the inner core NP from within the lower outer punch 5B into the upper outer punch 4B.

The manufacturing method of the press-coated fast-dissolving/disintegrating molded product of the present invention can be carried out using compression molding means having punches above and below a die, with at least the upper punch and preferably both the upper and lower punches having a double structure consisting of a center punch and an outer punch enclosing the outer perimeter of the center punch and both the center and outer punches being slidable and manipulatable for compression operation. Although a double-structured punch is normally employed for the lower punch as well as the upper punch, a normal punch may be used in some methods as with the aforementioned second example.

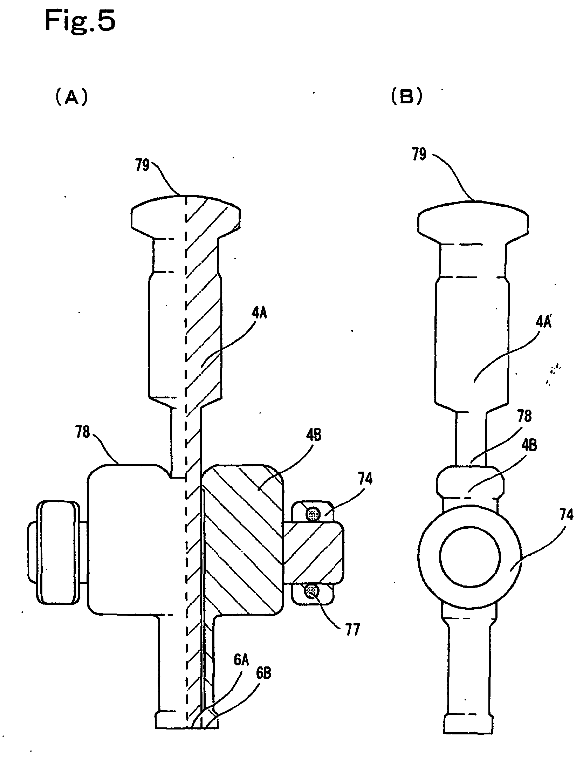

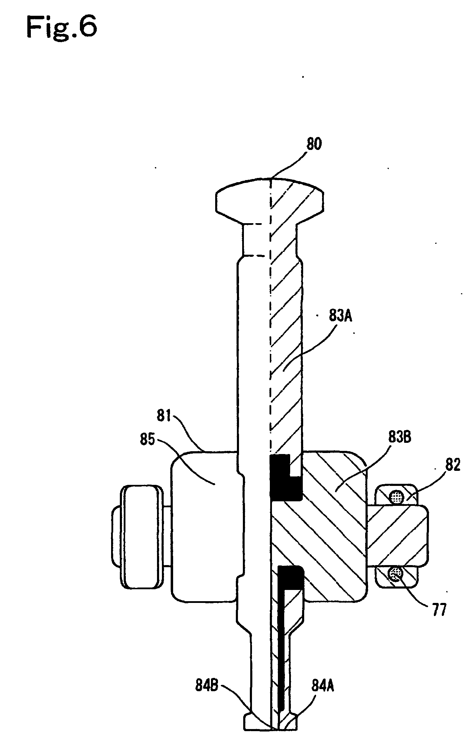

Thus, while a rotary compression molding machine of the present invention described later is among such compression molding means, the manufacturing method of the present invention can be basically readily performed by a hydraulic press, etc. as long as upper and lower punches and a die are available, with at least the upper punch having a double structure consisting of a center punch and an outer punch enclosing the outer perimeter of the center punch. That is, the manufacturing method of the present invention can be readily carried out by performing, in accordance with the sequence of steps of the present invention, a series of steps - steps of manually and/or automatically moving the upper and lower punches or the center and outer punches to predetermined positions, charging intended bulk materials (bulk materials for the outer layers and the inner core) and pressing the bulk materials so as to sandwich them from above and below in accordance with the sequence of steps of the present invention. It is to be noted that double-structured punches are normally used for the lower punch as well as the upper punch as mentioned earlier. For double-structured punches, refer to the description given later and Figs. 5 and 6.

The manufacturing method of the press-coated fast-dissolving/disintegrating molded product of the present invention can also be carried out using an apparatus for manufacturing the press-coated molded product of the present invention described below.

The apparatus for manufacturing the press-coated molded product of the present invention has adopted the basic parts of the mechanism and construction of previously known rotary compression molding machine, namely, the machine having a rotatable turntable, provided with a die having die holes and being designed to compress bulk materials charged into the die by holding upper and lower punches above and below the die so as to be vertically slidable, moving the upper and lower punches in mutually approaching directions and pressing the bulk materials with the punch tips left inserted in the die. Further, the apparatus comprises means for moving center and outer punches, with at least the upper punch having a double structure consisting of a center punch and the outer punch enclosing the outer perimeter of the center punch and both the center and outer punches being slidable and manipulatable for compression operation and means for allowing manipulation of the center and outer punches for compression operation and is constructed so as to perform a series of steps of the manufacturing method of the press-coated fast-dissolving/disintegrating molded product of the present invention.

That is, the apparatus for manufacturing the press-coated molded product of the present invention is a rotary compression molding machine characterized in having a rotatable turntable provided with a die having die holes and performing compression operation of bulk materials charged into the die by holding upper and lower punches above and below the die so as to be vertically slidable, moving the upper and lower punches in mutually approaching directions and pressing the bulk materials with the punch tips left inserted in the die, the machine using, for at least the upper punch, a double punch having a double structure consisting of a center punch and the outer punch enclosing the outer perimeter of the center punch, with both the center and outer punches being slidable and manipulatable for compression operation, and comprising means for moving the center and outer punches of the double punch and means for allowing manipulation of the center and outer punches for compression operation and the machine being provided, on the same turntable, with supply parts respectively for bulk materials for the outer layers and the inner core, compression molding parts for the bulk materials for the inner core and/or the outer layers and compression molding part for the entire molded product containing the inner core. There normally exist two or more supply parts for bulk materials for the outer layers in the present manufacturing apparatus.

It is to be noted that the apparatus for manufacturing the press-coated molded product of the present invention normally uses, also for the lower punch, a double punch having a double structure consisting of a center punch and the outer punch enclosing the outer perimeter of the center punch with both the center and outer punches being slidable and manipulatable for compression operation and comprises means for moving the center and outer punches of the double punch and means for allowing manipulation of the center and outer punches for compression operation.

Normally using bulk materials as molding materials, rotary compression molding machine may have a residual bulk material removal device of the present invention for removing bulk materials remaining on the lower outer punch and/or the molded product, depending on the shape of the punch tips.

A preferred embodiment of the apparatus for manufacturing the press-coated molded product of the present invention can be more specifically described as follows: Rotary compression molding machine characterized in using, for both the upper and lower punches, a double punch having a double structure consisting of a center punch and an outer punch enclosing the outer perimeter of the center punch, with both the center and outer punches being slidable and manipulatable for compression operation, comprising means for moving the center and outer punches of the double punches and means for allowing manipulation of the center and outer punches for compression operation and comprising a part for supplying a first bulk material into a space enclosed by the lower outer punch, a part for subsequent compression molding of the first bulk material by the upper and lower center punches, a part for supplying a second bulk material into a space enclosed by the lower outer punch, a part for subsequent compression molding of the second bulk material by the upper and lower center punches, a part for supplying a last bulk material into a space within the die and a part for subsequent compression molding of the entire molded product by the upper and lower center and outer punches.

To describe, in further detail, the apparatus for manufacturing the press-coated molded product of the present invention, descriptions will be given in succession beginning with conventional rotary compression molding machine.

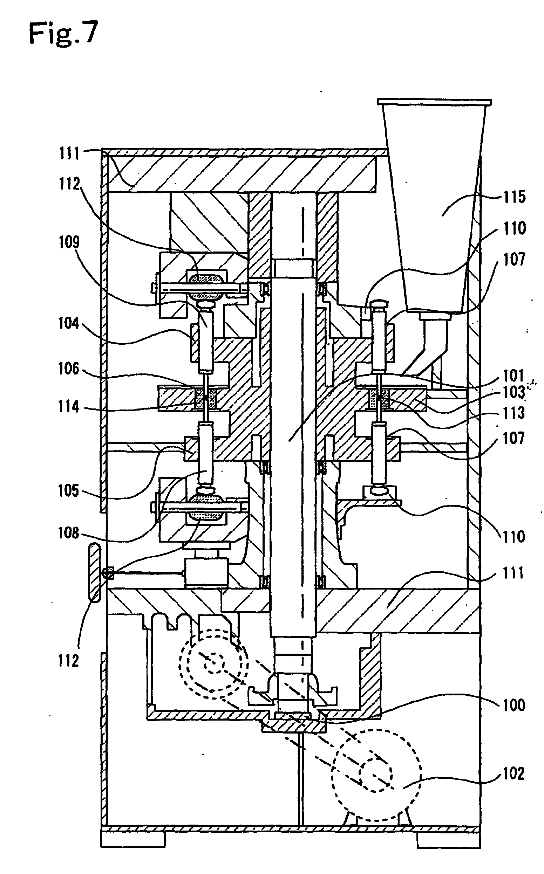

In case of shaft-driven, for example, rotary compression molding machine has a vertical shaft 101, supported by a bearing 100, arranged at the center part of a main body frame 111, with a motor 102 transmitting rotational drive force to the vertical shaft and a turntable 103 split into two functional parts, fixed near the vertical shaft, as shown in Fig. 7. Further, there are provided an upper punch holding part 104, located on the upper part of the turntable, for holding an upper punch so as to be vertically slidable and a lower punch holding part 105, located on the lower part of the turntable, for holding a lower punch so as to be vertically slidable such that the turntable 103 is sandwiched between the upper and lower punch holding parts 104 and 105. On the turntable 103, there is a die part made up of a plurality of die mounting holes 106, for fitting the die 114 so as to be detachable/reattachable, that are provided along the same circumference. On each of the upper and lower punch holding parts 104 and 105, there are a plurality of punch holding holes 107 drilled for holding the upper and lower punches so as to be slidable. Each of the punch holding holes 106 and the die mounting holes 107 is drilled on the turntable such that the lower punch 108, the upper punch 109 and the die 114 are arranged vertically with their center lines aligned. Tracks 110 are provided correspondingly for track contact parts of the upper punch 109 and the lower punch 108, and the punches move vertically on the tracks as they engage with and are guided by respective cams which will be discussed later. The die 114 has a die hole 113 cut vertically through the die into which the tips of the upper and lower punches 109 and 108 are inserted. It is to be noted that 112 represents a compression roller while 115 a hopper in Fig. 7.

In addition to shaft-driven rotary compression molding machine, there are other types thereof such as external gear-driven (external gear type) and internal gear-driven (internal gear type) rotary compression molding machines in which rotational drive force is transmitted by equipping the turntable with a gear.

Next, a description will be given of the double-structured punch used in the present invention and the parts associated therewith.

The double punch used in the present invention has a center punch and an outer punch enclosing the outer perimeter of the center punch, with the outer shape of the outer punch being approximately identical to the inner shape of a die and further the outer shape of the center punch being approximately identical to the outer shape of the inner core and the inner shape of the outer punch. Further, both the center and outer punches are slidable and manipulatable for compression operation. Here, the center and outer punches are basically slidable independently of each other, except for those parts that slide by coordination of the two punches.

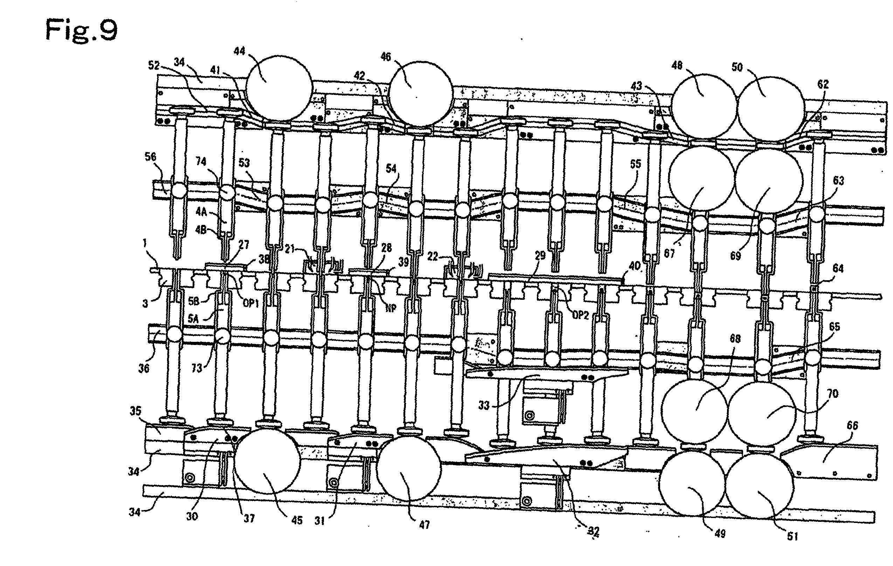

For example, the double punch is structured as shown in Figs. 5 that corresponds to Fig. 9 and has the center and outer punches 4A and 4B, an outer punch compression head 78, a center punch compression head 79 and an outer punch vertical sliding motion adjustment roller 74. In the compression molding step, compression of the center part of the tablet large in compression area is carried out by pressing the center punch compression head 79 with compression rollers (44, 46, 48, 50 in Fig. 9) whereas compression of the outer perimeter part of the tablet is performed by pressing the outer punch compression head 78 with compression rollers (67, 69 in Fig. 9). This allows for compression operation using the center and outer punches. Contact parts of the compression rollers of the center and outer punches (the outer punch compression head 78 and the center punch compression head 79) are vertically separated from each other, thus preventing interference between the compression rollers of the center and outer punches.

While the vertical sliding motion of the center punch is controlled by a normal method mainly using the center punch track and a center punch bottom part 37 (same part as the center punch compression head 79), the vertical sliding motion adjustment roller 74 is provided that comes in direct contact with the outer punch track to allow vertical sliding motion of the outer punch. Preferably, a plurality of bearings 77 are provided within the rollers to allow rotation of the rollers and smooth vertical sliding motion of the outer punch. Here, the vertical sliding motion adjustment roller 74 is arranged outside the outer punch compression head 78, with the vertical sliding motion adjustment roller 74 separated from the outer punch compression head 78. This allows the compression roller to apply pressure only to the outer punch compression head 78 while not applying direct pressure to the vertical sliding motion adjustment roller 74, thus preventing breakage of the bearings 77 within the vertical sliding motion adjustment roller 74. In compression operation, it is possible to apply pressure to the outer punch closer to the side of the center punch, thus allowing efficient transfer of pressure from the compression roller to the bulk materials.

While Figs. 5 assume the upper punch, the same holds true for the lower punch as long as a double-structured punch is used. Differences between the double-structured upper and lower punches are a longer length of the tip part of the lower punch inserted into the die and different parts prescribing the punch motion (e.g., spaces within the punches) due to the difference in motion between the upper and lower punches.

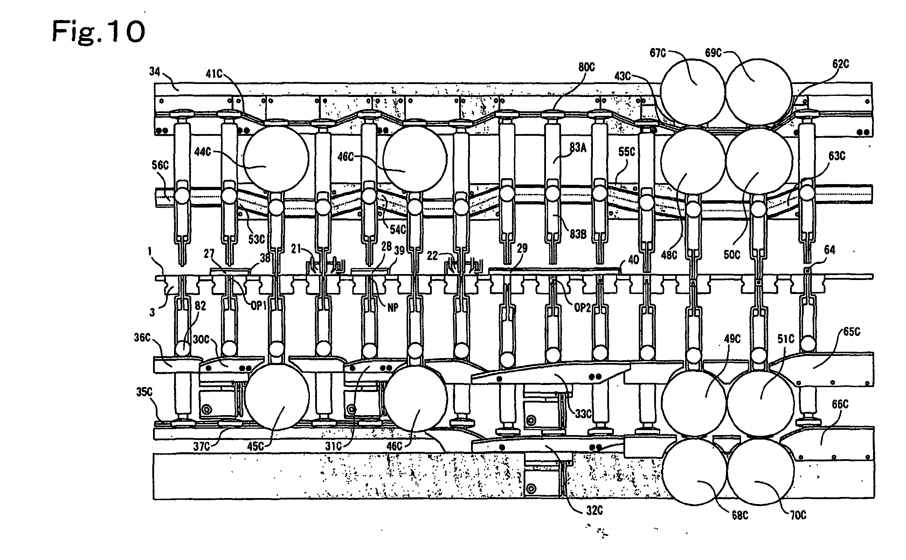

Alternatively, the double punch used in the present invention may be that corresponding to Fig. 10 in which the motions of the center and outer punches are respectively controlled in reverse. That is, the punch controls the motion of the center punch with the vertical sliding motion adjustment roller and the track and the motion of the outer punch with the punch bottom part (same part as the outer punch compression head 80) and the track. The punch is, as shown in Fig. 6, characterized in that an opening part (an outer punch opening part 85) is provided on the main body of the outer punch and that a center punch compression head 81 integral with the center punch and a center punch vertical sliding motion adjustment roller 82 projecting from the opening part. A description of the punch will be omitted since the same holds true for the punch as for the punch of Figs. 5, except that the motions of the center and outer punches are respectively controlled in reverse.

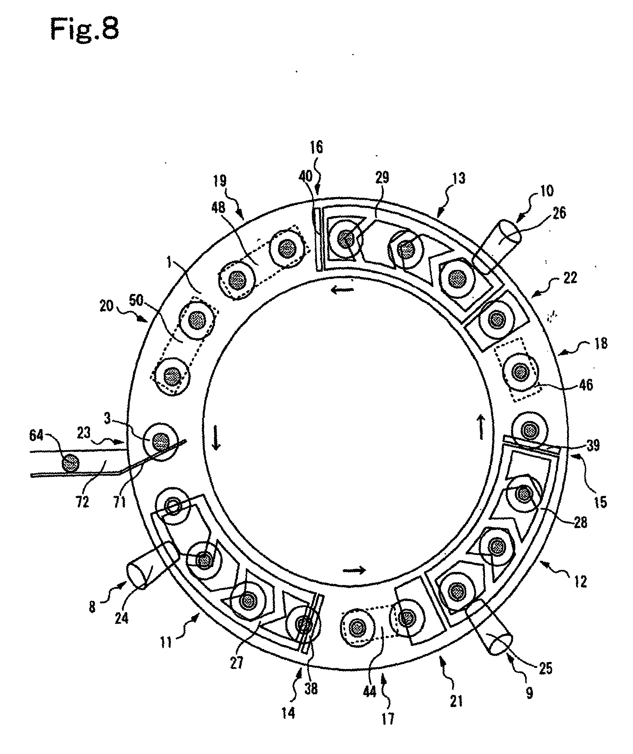

Next, a description will be given in detail of an embodiment of the apparatus corresponding to the first embodiment of the manufacturing method of the present invention (Figs. 1) together with operations of the parts thereof mainly with reference to Figs. 8 and 9 and, as necessary, Figs. 1 as the apparatus for manufacturing the press-coated molded product of the present invention that is the rotary compression molding machine. It is to be noted that bulk materials normally used as molding materials are employed in the embodiment.

When looked from above the turntable, bulk material supply parts 8, 9 and 10, bulk material charging parts 11, 12 and 13, bulk material rubbing-cutting parts 14, 15 and 16, compression molding parts 17, 18, 19 and 20, residual bulk material removal parts 21 and 22 and a product edjecting part 23 are provided along the direction of rotation of a turntable 1 as shown in Fig. 8.

Individual mechanisms will be described separately. The bulk material supply parts (8, 9, 10 in Fig. 8) can be separated, according to the sequence of supply of bulk materials, into the part 8 for supplying the bulk material for the first outer layer OP1, the part 9 for supplying the bulk material for the inner core NP and the part 10 for supplying the bulk material for the second outer layer OP2, with the bulk materials supplied from hoppers 24, 25 and 26 filled with the respective bulk materials by natural fall or by a metered supply machine (not shown).

The respective bulk materials supplied by the bulk material supply parts are sent next to the bulk material charging parts (11, 12, 13 in Fig. 8) . The bulk material charging parts are designed to charge each of the bulk materials for the first outer layer OP1, the inner core NP and the second outer layer OP2, respectively into the first outer layer space 201A, the inner core space 202A or the second outer layer space 203A (refer to Figs. 1) . These parts are intended to hold fixed amounts of the respective bulk materials supplied from the bulk material supply parts using open feed shoes 27, 28 and 29, provided on the turntable 1 and capable of both storing and supplying the bulk materials, and introduce each of the bulk materials held by the feed shoes 27, 28 and 29 into the first outer layer space 201A, the inner core space 202A or the second outer layer space 203A (refer to Figs. 1) by lowering the lower center punch 5A using lowerers 30, 31 and 32 provided on a frame 34, and in certain circumstances, by lowering the lower outer punch 5B using a lowerer 33 provided on a lower outer punch track 36. Although the third open feed shoe 29 is shown larger than the other open feed shoes in Fig. 9, this is intended to provide a detailed description thereof. It is to be noted that, in place of the open feed shoes, agitation feed shoes may be employed that forcefully charge the bulk materials into the die using agitation vanes (installed at the same positions as the open feed shoes; not shown).

In detail, the bulk material for the first outer layer OP1 is charged by lowering the lower center punch 5A within the first open feed shoe 27 on the turntable 1 (Figs. 1A and 1B) . Here, the lower outer punch 5B is maintained at a constant height with respect to the turntable by moving the lower outer punch 5B on the lower outer punch track 36 installed so as to bring the extreme tip part of the lower outer punch 5B to the same height as the surface of the turntable 1 using the vertical sliding motion adjustment roller 73 of the lower outer punch. On the other hand, the lower center punch 5A is moved on a lower center punch track 35 provided on the frame 34 using the lower center punch bottom part 37 (substantially same part as the center punch compression head 79 in Figs. 5) and further adjusted to a predetermined position using the first center punch lowerer 30 provided on the lower center punch track 35. The bulk material for the first outer layer OP1 is thus introduced into the first outer layer space 201A enclosed by the lower outer punch 5B and above the lower center punch 5A.

Next, the bulk material for the inner core NP is charged by lowering only the lower center punch 5A within the second open feed shoe 28 on the turntable 1 as with the first outer layer OP1 (Figs. 1E and F). Here, the lower outer punch 5B is maintained at a constant height with respect to the turntable by moving the lower outer punch 5B on the lower outer punch track 36 installed so as to bring the extreme tip part of the lower outer punch 5B to the same height as the surface of the turntable 1 using the vertical sliding motion adjustment roller 73 of the lower outer punch. On the other hand, the lower center punch 5A holding the temporary molded product of the first outer layer on a lower center punch upper end surface 7A is moved on the lower center punch track 35 provided on the frame 34 using the lower center punch bottom part 37 and further lowered using the second center punch lowerer 31 provided on the lower center punch track 35. The bulk material for the inner core NP is thus introduced into the inner core space 202A enclosed by the lower outer punch 5B and above the temporary molded product of the first outer layer.

Further, the bulk material for the second outer layer OP2 is charged by lowering both the lower center punch 5A holding the temporarily molded first outer layer OP1 and inner core NP and the lower outer punch 5B or only the lower outer punch 5B within the third open feed shoe 29 on the turntable 1 (Figs. 1I and 1J) . Here, the lower outer punch 5B is lowered using the lower outer punch lowerer 33 provided on the lower outer punch track 36. On the other hand, the lower center punch 5A is moved on the lower center punch track 35 provided on the frame 34 using the lower center punch bottom part 37 and lowered using the third center punch lowerer 32 provided on the lower center punch track 35. The bulk material for the second outer layer OP2 is thus introduced into the second outer layer space 203A created above and around the temporary molded products of the first outer layer OP1 and the inner core NP within the die 3 by lowering both the lower center and outer punches 5A and 5B or only the lower outer punch 5B.

The die and punches charged with the bulk materials by the bulk material charging parts next enter the bulk material rubbing-cutting parts (14, 15, 16 in Fig. 8). The bulk material rubbing-cutting parts adjust each bulk materials for the first outer layer OP1, the inner core NP and the second outer layer OP2 supplied and charged as described above to fixed amounts. That is, the respective excess bulk materials overflowing from the given spaces are rubbed and cut for removal by rubbing-cutting plates 38, 39 and 40 as the lower center punch 5A or both the lower center and outer punches 5A and 5B are raised to predetermined positions using the lower outer punch track 36 and the lower center punch track 35.

In detail, the bulk material for the first outer layer OP1 is rubbed and cut by the rubbing-cutting plate 38 attached to the first open feed shoe 27 on the turntable 1. With the extreme tip part of the lower outer punch 5B level with the surface of the turntable 1, the lower center punch 5A is raised to a predetermined position, thus causing the excess amount of the bulk material for the first outer layer OP1 charged into the first outer layer space 201A to overflow from the space. Further, the overflowing bulk material for the first outer layer OP1 is rubbed and cut by the rubbing-cutting plate 38 attached to the open feed shoe 27, thus leaving behind a fixed amount of the charged bulk material for the first outer layer OP1 (prior to Fig. 1B).

Next, the bulk material for the inner core NP is rubbed and cut by the rubbing-cutting plate 39 attached to the second open feed shoe 28 on the turntable 1 as with the bulk material for the first outer layer. Here, with the extreme tip part of the lower outer punch 5B level with the surface of the turntable 1, the lower center punch 5A is raised to a predetermined position, thus causing the excess amount of the bulk material for the inner core NP charged into the inner core space 202A to overflow from the space. Further, the overflowing bulk material for the inner core NP is rubbed and cut by the rubbing-cutting plate 39 attached to the second open feed shoe 28, thus leaving behind a fixed amount of the charged bulk material for the inner core NP (prior to Fig. 1F).

The bulk material for the second outer layer OP2 is rubbed and cut by the rubbing-cutting plate 40 attached to the third open feed shoe 29 on the turntable 1 as with the bulk materials for the first outer layer and the inner core. Here, the temporary molded products of the first outer layer and the inner core held by the lower center and outer punches 5A and 5B are pushed up into the bulk material for the second outer layer OP2 supplied into the die 3 as the lower center punch 5A or both the lower center and outer punches 5A and 5B are raised to predetermined positions, thus causing the excess amount of the bulk material for the second outer layer OP2 to overflow. Further, the overflowing bulk material for the second outer layer OP2 is rubbed and cut by the rubbing-cutting plate 40 attached to the third open feed shoe 29, thus leaving behind a fixed amount of the charged bulk material for the second outer layer OP2 (following Fig. 1K).

The die and punches charged with fixed amounts of the bulk materials next enter the compression molding parts (17, 18, 19, 20 in Fig. 8). Thecompressionmolding parts are intended to perform precompression or main compression on one of the bulk materials for the first outer layer OP1, the inner core NP and the second outer layer OP2 or a combination of two or more thereof (including temporary molded products) using compression rollers (44 to 51, 67 to 70) held by the frame 34.

In detail, precompression of the bulk material for the first outer layer OP1 or the bulk materials for the first outer layer OP1 and the inner core NP is carried out by pressing using the upper and lower center punches 4A and 5A. Here, the upper center punch 4A is lowered by upper center punch lowering cams 41 and 42 furnished on an upper center punch track 52, and preferably the upper outer punch 4B is also concurrently lowered to a predetermined position by upper outer punch lowering cams 53 and 54 furnished on an upper outer punch track 56, thus inserting the tip of the upper center punch 4A into the space above the lower center punch 5A and enclosed by the lower outer punch 5B within the die 3. The bulk material for the first outer layer OP1 charged into the given space or the temporary molded product of the first outer layer OP1 and the bulk material for the inner core NP are thus confined from above and below and pressed so as to be sandwiched between the upper temporary compression rollers 44 and 46 and the lower temporary compression rollers 45 and 47, thus molding a temporary molded product (Figs. 1C and 1G).

Precompression (temporary compression) of the temporary molded products of the first outer layer OP1 and the inner core NP and the bulk material for the second outer layer OP2 is carried out by pressing using the upper center and outer punches 4A and 4B (upper punch) and the lower center and outer punches 5A and 5B (lower punch) . To insert the upper center and outer punches 4A and 4B into the die 3, the upper center and outer punches 4A and 4B are lowered to predetermined positions using an upper center punch lowering cam 43 furnished on an upper center punch track 52 and an upper outer punch lowering cam 55 furnished on the upper outer punch track 56, inserting the tips thereof into the die 3. The temporary molded products of the first outer layer OP1 and the inner core NP and the bulk material for the second outer layer OP2 are confined so as to be sandwiched from above and below and press-molded in a preliminary fashion by the precompression roller 48 for the upper center punch, the precompression roller 67 for the upper outer punch, a precompression roller 49 for the lower center punch and the precompression roller 68 for the lower outer punch.

In main compression following precompression (temporary compression), the molded product press-molded in a preliminary fashion is press-molded as is finally by the main compression roller 50 for the upper center punch, the main compression roller 69 for the upper outer punch, the main compression roller 51 for the lower center punch and the main compression roller 70 for the lower outer punch (Fig. 1M). It is to be noted that although not preferred, it is possible to use only the present main compression part by omitting the precompression part of the molded products of the first outer layer OP1 and the inner core NP and the bulk material for the second outer layer OP2.

Next, the residual bulk material removal parts (21, 22 in Fig. 8) are provided at the precompression part of the bulk material for the first outer layer OP1 or the inner core NP or a part immediately thereafter. As shown in Figs. 1, in the temporary compression process or immediately thereafter, the lower outer punch 5B is held such that the extreme tip part thereof is maintained at the same height as the surface of the turntable 1, and preferably the space within the lower outer punch 5B is covered by the upper center punch 4A, thus removing the bulk material 57A for the first outer layer OP1 or the bulk material 57B for the inner core NP remaining on the upper end surface 7B of the lower outer punch by compressed air injection and suction, etc.

In detail, the upper end surface 7B of the lower outer punch 5B shown in Figs. 1 corresponds to the circumferential edge 76 of the finished product shown in Figs. 12, and the residual bulk materials 57 (57A, 57B) remain at the part. The residual bulk materials 57 are impossible to remove by rubbing and cutting using the rubbing-cutting plates 38 and 39 of the open feed shoes or agitation feed shoes provided on the turntable 1 and, if left unremoved, cause a concern over contamination between the bulk materials for the first outer layer OP1 and the inner core NP and that between the bulk materials for the inner core NP and the second outer layer OP2. In the present embodiment, for this reason, the residual bulk materials 57 (57A, 57B) are removed by the first and second residual bulk material removal parts 21 and 22 furnished on the turntable 1 following the precompression step (Fig. 1D and 1H). A residual bulk material removal device constituting the residual bulk material removal part comprises, for example as shown in Figs. 11, compressed air injection nozzles 60 for injecting compressed air onto the die surface from all directions and suction boxes 58 and 61 provided with suction holes 59 for sucking the residual bulk materials, with the compressed air injection nozzles 60 and the suction boxes 58 and 61 arranged on and parallel with the surface of the turntable 1 so as to sandwich the die and the punches. The compressed air injection nozzles 60 inject compressed air onto the punches and the die from all directions and further the suction holes 59 near the die surface aspirate the residual bulk materials 57, keeping the residual bulk materials from flying outside for reliable removal thereof.

Alternative method of removing the residual bulk materials is by raising the upper center punch 4A or the upper center and outer punches 4A and 4B with the temporary molded product held in the space inside the lower outer punch 5B and aspirating the entire die from the upper end surface of the die (from the direction perpendicular to the turntable), thus removing the bulk material for the first outer layer OP1 or the inner core NP remaining on the upper end surface 7B of the lower outer punch and/or the temporary molded product. It is mandatory in the method that the temporary molded product not be aspirated by suction, and remporary compression operation cannot be eliminated from the outer layer molding step and the outer layer/inner core molding step. These residual bulk material removal devices described above are the residual bulk material removal devices of the present invention. It is to be noted that the present residual bulk material removal parts may be omitted under certain circumstances. In particular, when a flat-surfaced molded product is made, the outer punch surface is also flat, thus requiring no residual bulk material removal parts.

The final molded product is sent to the product ejecting part (23 in Fig. 8) for ejection outside the molding apparatus. The product ejecting part is designed to eject the product using a scraper 71 that guides to a chute 72 by pushing up the product as the lower center and outer punches 5A and 5B rise.

In detail, the upper center and outer punches 4A and 4B are raised along the rising sloped surface by upper center and outer punch raising cams 62 and 63, thus pulling the punch tips out of the die 3. Further, using lower center and outer punch push-up rails 66 and 65, the lower center and outer punches 5A and 5B are pushed up, thus completely pushing a molded product 64 out of the die 3. Here, it is preferred for easy ejecting of the molded product that the tip surface of the lower outer punch 5B be maintained at the same height as the surface of the turntable 1 and that the lower center punch 5A be pushed up slightly more upward than the tip surface of the lower outer punch 5B (Fig. 1N) . The molded product 64 that has been pushed out is scraped using the scraper 71 for ejecting outside the turntable 1 and then guided into the chute 72 for ejection of the product.

In the apparatus of the present invention shown in Fig. 9, means for moving the center and outer punches refer to the tracks (the lower outer punch track 36, the lower center punch track 35, the upper outer punch track 56, the upper center punch track 52), the lowerers (the first center punch lowerer 30, the second center punch lowerer 31, the third center punch lowerer 32, the lower outer punch lowerer 33), the raising cams (the upper center and outer punch raising cams 62 and 63), the lowering cams (the upper center punch lowering cams 41, 42 and 43, the upper outer punch lowering cams 53, 54 and 55), the push-up rails (the lower center and outer punch push-up rails 66 and 65), the vertical sliding motion adjustment rollers (the vertical sliding motion adjustment rollers 73 and 74 of the lower and upper outer punches) , the lower center punch bottom part 37 and the bearings 77. On the other hand, means for allowing manipulation of the center and outer punches for compression operation refer to the compression rollers (the upper temporary compression rollers 44 and 46, the lower temporary compression rollers 45 and 47, the precompression roller 48 for the upper center punch, the precompression roller 67 for the upper outer punch, the precompression roller 49 for the lower center punch, the precompression roller 68 for the lower outer punch, the main compression roller 50 for the upper center punch, the main compression roller 69 for the upper outer punch, the main compression roller 51 for the lower center punch, the main compression roller 70 for the lower outer punch) , and the outer punch compression head 78 and center punch compression head 79 in Figs. 5. It is to be noted that these include not only elements of the apparatus main body but also those of the punches.

As already described in relation to the punches, means for moving the center and outer punches or means for allowing manipulation of the center and outer punches for compression operation include not only the method as shown in Fig. 9 of controlling the motions of the outer punches by the vertical sliding motion adjustment rollers and tracks thereof and the motions of the center punches by the center punch bottom parts and tracks thereof (corresponding to the punches in Figs. 5) but also an alternative method as shown in Fig. 10 that is a reverse thereof in which the motions of the center punches are controlled by the vertical sliding motion adjustment rollers and tracks and the motions of the outer punches by the punch bottom parts and tracks. It is to be noted that while Fig. 10 corresponds to the first example (Figs. 1) of the manufacturing method of the press-coated fast-dissolving/disintegrating molded product of the present invention as Fig. 9 does, some parts are named differently due to the fact that the punch motions are controlled in reverse as compared with Fig. 9. However, since the basic operational mechanism remains unchanged from Fig. 9, the description of signs, names, etc. is omitted. For those parts in Fig. 10 that are named differently from Fig. 9 due to reverse control, "C" is added to the signs for the corresponding parts.

When a fast-dissolving/disintegrating molded product having a plurality of cores is made using the apparatus for manufacturing the press-coated molded product of the present invention, parts for handling from supply to molding of inner cores and outer layers are increased, under certain circumstances, together with residual bulk material removal parts on the same turntable in accordance with the number of inner cores and that of outer layer parts separating the inner cores. That is, in the case of the turntable shown in Fig. 8, as many parts as necessary are added for handling supply to molding of inner cores and outer layers together with residual bulk material removal parts.

The apparatus used in the second example (Figs. 2) of the manufacturing method of the press-coated fast-dissolving/disintegrating molded product of the present invention is basically similar to that described in detail in the first example. The differences therebetween are that, correspondingly with the difference in punch motions and compression operation, means for moving the punches, compression means and so on are naturally different and that there is only one residual bulk material removal part. It is to be noted that the apparatus used in the third example of the manufacturing method of the press-coated fast-dissolving/disintegrating molded product of the present invention can be made in conformance with the first and second examples.

The press-coated fast-dissolving/disintegrating molded product according to the present invention will be described below with reference to embodiments. It is to be noted that in Test Example 1, the dissolubility or/and disintegrability of a molded product was evaluated by molding ingredients rich in dissolubility or/and disintegrability (mainly saccharides or/and sugar alcohols) to be added to the inner core.

### Test Example 1

### [Method]

The rate of 79.5 in 80 of erythritol (NIKKEN CHEMICALS) and 0.5 in 80 of magnesium stearate (TAIHEI CHEMICAL INDUSTRIAL) were mixed. Next, 80mg of the mixture was weighed into a space enclosed by a lower punch and the inner wall of a die in an apparatus equipped with the die and upper and lower punches of 6.0mm in diameter manipulatable for pressing operation and having flat edge angle. Then, by moving the upper and lower punches in mutually approaching directions and using a hydraulic manual press (Iuchi Seieido: 3t high-pressure jack), tabletting was carried out at a compression pressure of about 500kg/tablet, thus producing a molded product for use as a sample. Molded products were similarly made for mannitol (TOWA CHEMICAL INDUSTRY), xylitol (TOWA CHEMICAL INDUSTRY), lactose (DMV) and xylose (TOWA CHEMICAL INDUSTRY).

The dissolubility or/and disintegrability of the molded products made as samples were measured for evaluation in accordance with the Disintegration Test of the General Tests, Processes and Apparatus of the Japanese Pharmacopoeia, Fourteenth Edition using a disintegration tester (TOYAMA CHEMICAL) and water as test liquid in the presence of an auxiliary table. The disintegration time in an actual human oral cavity was also measured. The results are shown in Table 1.

### [Results and Discussion]

In the case of erythritol, the disintegration time according to the Japanese Pharmacopoeia was 5 seconds whereas that in the oral cavity 3 seconds. The results for the others are as shown in Table 1. In Table 1, erythritol, mannitol and xylitol are sugar alcohols whereas lactose and xylose saccharides. Particularly excellent in dissolubility or/and disintegrability were erythritol and xylose.

From the aforementioned results, erythritol and xylose were selected for Test Example 2 as ingredients rich in dissolubility or/and disintegrability (mainly saccharides or/and sugar alcohols) to be added to the inner core, and the effect was evaluated of dissolution/disintegration accelerant added to the inner core on the dissolubility or/and disintegrability using erythritol and xylose.

**Table 1**

| Composition of samples | Tabletting pressure | Tablet weight | Disintegration time (Japanese Pharmacopoeia) | Disintegration time (oral cavity) |
|---|---|---|---|---|
| Erythrito 1 79.5mg Magnesium stearate 0.5mg | 500kg/tab let | 80mg | 5 sec. | 3 sec. |
| Mannitol 79.5mg Magnesium stearate 0.5mg | 500kg/tab let | 80mg | 17 sec. | 8 sec. |
| Xylitol 79.5mg Magnesium stearate 0.5mg | 500kg/tab let | 80mg | 10 sec. | 12 sec. |
| Lactose 79.5mg Magnesium stearate 0.5mg | 500kg/tab let | 80mg | 21 sec. | 12 sec. |
| Xylose 79.5mg Magnesium stearate 0.5mg | 500kg/tab let | 80mg | 5 sec. | 4 sec. |

### Test Example 2

### [Method]

The rate of 72 in 80 of erythritol (NIKKEN CHEMICALS), 7.5 in 80 of corn starch (NIHON SHOKUHIN KAKO) and 0.5 in 80 of magnesium stearate (TAIHEI CHEMICAL INDUSTRIAL) were mixed. Next, 80mg of the mixture was weighed into a space enclosed by a lower punch and the inner wall of a die in an apparatus equipped with the die and upper and lower punches of 6.0mm in diameter manipulatable for pressing operation and having flat edge angle. Then, by moving the upper and lower punches in mutually approaching directions and using a hydraulic manual press (same as above) , tabletting was carried out at a compression pressure of about 2000kg/tablet, thus producing a molded product for use as a sample. Separately therefrom, the rate of 79.5 in 80 of erythritol (NIKKEN CHEMICALS) and 0.5 in 80 of magnesium stearate (TAIHEI CHEMICAL INDUSTRIAL) were mixed, similarly producing a molded product. For xylose (TOWA CHEMICAL INDUSTRY), two types of molded products were similarly made.

The molded products made as samples were evaluated for dissolubility or/and disintegrability in the same manner as with Test Example 1. The results are as shown in Table 2.

### [Results and Discussion]

In the case of erythritol, the disintegration time according to the Japanese Pharmacopoeia was five seconds whereas that in the oral cavity seven seconds. The disintegration times according to the Japanese Pharmacopoeia and in the oral cavity were respectively reduced to 1 second and three seconds as a result of blending of approximately 10% of corn starch as a dissolution/disintegration accelerant. The results for xylose are as shown in Table 2. It was discovered from the above that addition of a dissolution/disintegration accelerant such as corn starch to the inner core provides improved dissolubility or/and disintegrability. Although not shown here, this tendency was prominent at a high tabletting pressure (2000kg/tablet) rather than at a relatively low pressure (500kg/tablet).

From the aforementioned results, a 9:1 blending ratio of erythritol (ingredient rich in dissolubility or/and disintegrability) to corn starch (dissolution/disintegration accelerant) was selected for Test Example 3 for addition to the inner core, and the effect was evaluated of erythritol particle size on the dissolubility or/and disintegrability using the ratio.

**Table 2**

| Composition of samples | Tabletting pressure | Tablet weight | Disintegration time (Japanese Pharmacopoeia) | Disintegration time (oral cavity) |
|---|---|---|---|---|
| Erythritol 72mg Corn starch 7.5mg Magnesium stearate 0.5mg | 2000kg/tablet | 80mg | 1 sec. | 3 sec. |
| Erythritol 79.5mg Magnesium stearate 0.5mg | 2000kg/tablet | 80mg | 5 sec. | 7 sec. |
| Xylose 72mg Corn starch 7.5mg Magnesium stearate 0.5mg | 2000kg/tablet | 80mg | 6 sec. | 2 sec. |
| Xylose 79.5mg Magnesium stearate 0.5mg | 2000kg/tablet | 80mg | 64 sec. | 8 sec. |

### Test Example 3

### [Method]

The rate of 72 in 80 of erythritol of various particle sizes (NIKKEN CHEMICALS), 7.5 in 80 of corn starch (NIHON SHOKUHIN KAKO) and 0 . 5 in 80 of magnesium , stearate (TAIHEI CHEMICAL INDUSTRIAL) were mixed. Next, 80mg of the mixture was weighed into a space enclosed by a lower punch and the inner wall of a die in an apparatus equipped with the die and upper and lower punches of 6.0mm in diameter manipulatable for pressing operation and having flat edge angle. Then, by moving the upper and lower punches in mutually approaching directions and using a hydraulic manual press (same as above), tabletting was carried out at a compression pressure of about 2000kg/tablet, thus producing a molded product for use as a sample. It is to be noted that erythritol was screened in advance to particle sizes as shown in Table 3 before use.

The molded products made as samples were evaluated for dissolubility or/and disintegrability in the same manner as with Test Example 1. The results are as shown in Table 3.

### [Results and Discussion]

Erythritol exhibited excellent dissolubility or/and disintegrability (seven seconds or less in the Disintegration Test of the Japanese Pharmacopoeia) for a particle size from 75 to 710µm (22 to 200 mesh) under a high-pressure tabletting condition (2000kg/tablet). Erythritol particle size is preferably 150 to 710µm (22 to 100 mesh). Although not shown here, this tendency was prominent at a high tabletting pressure (2000kg/tablet) rather than at a relatively low pressure (500kg/tablet).

From the aforementioned results, erythritol (particle size: 150 to 710µm) and corn starch were selected for the inner core respectively as ingredient rich in dissolubility or/and disintegrability and dissolution/disintegration accelerant, with erythritol and corn starch blended at a 9-to-1 ratio for Test Example 4. The effect was evaluated of ingredients rich in moldability added to the outer layers on the dissolubility or/and disintegrability in the case of such an inner core. All erythritol used in successive Test Examples 4 to 9 was prepared by screening to particle size from 150 to 710µm (22 to 100 mesh), and the description of its particle size is omitted.

**Table 3**

| Erythritol particle size | Tabletting pressure | Tablet weight | Disintegration time (Japanese Pharmacopoeia) | Disintegration time (oral cavity) |
|---|---|---|---|---|
| 355-710µm (22-42 mesh) | 2000kg/tab let | 80mg | 4 sec | 5 sec |
| 250-355µm (42-60 mesh) | 2000kg/tab let | 80mg | 2 sec | 5 sec |
| 150-250µm (60-100 mesh) | 2000kg/tab let | 80mg | 3 sec | 7 sec |
| 75-150µm (100-200 mesh) | 2000kg/tab let | 80mg | 7 sec | 7 sec |
| 75µm or less (200 mesh pass) | 2000kg/tab let | 80mg | 17 sec | 10 sec |
| Sample composition: 72mg of erythritol, 7.5mg of corn starch, 0.5mg of magnesium stearate | | | | |

### Test Example 4

### [Method]

The rate of 72 in 80 of erythritol (NIKKEN CHEMICALS), 7.5 in 80 of corn starch (NIHON SHOKUHIN KAKO) and 0.5 in 80 of magnesium stearate (TAIHEI CHEMICAL INDUSTRIAL) were mixed for use as bulk material for the inner core. Separately therefrom, the rate of 79.5 in 80 of crystalline cellulose (Asahi KASEI) and 0.5 in 80 of magnesium stearate (TAIHEI CHEMICAL INDUSTRIAL) were mixed for use as bulk material for the outer layer. 20mg of the bulk material for the outer layer was weighed into a space above a lower center punch and enclosed by a lower outer punch, with the lower center punch among the pressing punches lowered, in an apparatus equipped with a die and upper and lower punches of 6.0mm in inner diameter and 8.0mm in outer diameter having a double structure, manipulatable for pressing operation and having flat edge angle. Then, by moving the upper and lower center punches in mutually approaching directions, temporary compression was manually conducted to such an extent that the surface became flat. Next, 80mg of the bulk material for the inner core was weighed into a space enclosed by the lower outer punch and above the temporary molded product of the outer layer, and precompression was manually conducted in the same manner as above by moving the upper and lower center punches in mutually approaching directions. Further, the remaining 60mg of bulk material for the outer layer was weighed into a space enclosed by the inner wall of the die and above the temporary molded products of the outer layer and the inner core such that the temporary molded product of the inner core was completely covered by the bulk material for the outer layer. Then, by moving the upper and lower punches in mutually approaching directions, tabletting was conducted at a compression pressure of about 500kg/tablet - this time using a hydraulic manual press (same as above) . Molded products were made similarly for hydroxypropylcellulose (NIPPON SODA) and sorbitol (NIKKEN CHEMICALS). The molded products made as samples were evaluated for dissolubility or/and disintegrability in the same manner as with Test Example 1. As for moldability evaluation, the tablet weight was measured after 100 revolutions using a motor-equipped drum adjusted to 24 to 26 revolutions per minute in conformance with reference information "Tablet Friability Test" in the Japanese Pharmacopoeia, Fourteenth Edition. Degree of friability was determined by calculating the percentage of decline in weight relative to the tablet weight at the beginning of the test. The results are shown in Table 4.

### [Results and Discussion]

The molded products as a whole using crystalline cellulose, hydroxypropylcellulose and sorbitol were all found to exhibit 0% friability - excellent moldability. On the other hand, while the disintegration time according to the Japanese Pharmacopoeia was over 60 seconds for hydroxypropylcellulose and sorbitol - poor dissolubility or/and disintegrability, crystalline cellulose excelled in dissolubility or/and disintegrability as well with the disintegration time of 44 seconds.

From the aforementioned results, crystalline cellulose also rich in dissolubility or/and disintegrability was selected as ingredient rich in moldability to be added to the outer layer for Test Example 5, and crystalline cellulose was blended with an ingredient rich in dissolubility or/and disintegrability for evaluation of the effect imparted to the moldability and dissolubility or/and disintegrability. As ingredients rich in dissolubility or/and disintegrability, erythritol, mannitol and lactose were selected from among those that exhibited excellent dissolubility or/and disintegrability in the inner core design stage (Test Example 1).

**Table 4**

| Outer layer composition of samples | Tabletting pressure | Tablet weight | Disintegration time (Japanese Pharmacopoeia) | Disintegration time (oral cavity) | Friability (100 rev.) |
|---|---|---|---|---|---|
| Crystalline cellulose 79.5mg Magnesium stearate 0.5mg | 500kg/tabl et | 160mg | 44 sec. | 22 sec. | 0.00% |
| Hydroxypropylcellul ose 79.5mg Magnesium stearate 0.5mg | 500kg/tabl et | 160mg | Over 1800 sec. | 362 see. | 0.00% |
| Sorbitol 79.5mg Magnesium stearate 0 .5mg | 500kg/tabl et | 160mg | 145 sec. | 33 sec. | 0.00% |
| Sample's inner core composition: 72mg of erythritol, 7.5mg of corn starch, 0.5mg of magnesium stearate Test Example 5 | | | | | |

### [Method]

The rate of 72 in 80 of erythritol (NIKKEN CHEMICALS), 7.5 in 80 of corn starch (NIHON SHOKUHIN KAKO) and 0.5 in 80 of magnesium stearate (TAIHEI CHEMICAL INDUSTRIAL) were mixed for use as bulk material for the inner core. Separately therefrom, the rate of 19.5 in 80 of crystalline cellulose (Asahi KASEI), 60 in 80 of erythritol and 0.5 in 80 of magnesium stearate (TAIHEI CHEMICAL INDUSTRIAL) were mixed for use as bulk material for the outer layer. Next, 20mg of the bulk material for the outer layer was weighed into a space above a lower center punch and enclosed by a lower outer punch, with the lower center punch among the pressing punches lowered, in an apparatus equipped with a die and upper and lower punches of 6.0mm in inner diameter and 8.0mm in outer diameter having a double structure, manipulatable for pressing operation and having flat edge angle. Then, by moving the upper and lower center punches in mutually approaching directions, temporary compression was manually conducted to such an extent that the surface became flat. Next, 80mg of the bulk material for the inner core was weighed into a space enclosed by the lower outer punch and above the temporary molded product of the outer layer, and temporary compression was manually conducted in the same manner as above by moving the upper and lower center punches in mutually approaching directions. Further, the remaining 60mg of bulk material for the outer layer was weighed into a space enclosed by the inner wall of the die and above the temporary molded products of the outer layer and the inner core such that the temporary molded product of the inner core was completely covered by the bulk material for the outer layer. Then, by moving the upper and lower punches in mutually approaching directions, tabletting was conducted at a compression pressure of about 500kg/tablet - this time using a hydraulic manual press (same as above). Molded products were made similarly at the same blending ratio for the outer layer shown in Table 5 using mannitol (TOWA CHEMICAL INDUSTRY) and lactose (DMV) in place of erythritol for the outer layer.

The molded products made as samples were evaluated for dissolubility or/and disintegrability in the same manner as with Test Example 1. Moldability evaluation was conducted in the same manner as with Test Example 4. The results are shown in Table 5.

### [Results and Discussion]

The molded products as a whole were found to exhibit friability of 0.62% or less and a disintegration time of 13 seconds or less according to the Japanese Pharmacopoeia which was excellent both in moldability and dissolubility or/and disintegrability, as a result of blending in the outer layer of crystalline cellulose as ingredient rich in moldability and erythritol, mannitol and lactose as ingredients rich in dissolubility or/and disintegrability.

From the aforementioned results, crystalline cellulose was selected as ingredient rich in moldability and erythritol as ingredient rich in dissolubility or/and disintegrability to be added to the outer layer for Test Example 6, and the effect was evaluated of the blending ratio thereof on the moldability and dissolubility or/and disintegrability. Similarly, the ingredient rich in dissolubility or/and disintegrability to be added to the outer layer was changed to mannitol in Test Example 7 and to lactose in Test Example 8 for evaluation.

**Table 5**

| Outer layer composition of samples | Tabletting pressure | Tablet weight | Disintegration time (Japanese Pharmacopoeia) | Disintegration time (oral cavity) | Friability (100 rev.) |
|---|---|---|---|---|---|
| Erythritol 40mg Crystalline cellulose 39.5mg Magnesium stearate 0.5mg | 500kg/tablet | 160mg | 11 sec. | 8 sec. | 0.62% |
| Mannitol 50mg Crystalline cellulose 29.5mg Magnesium stearate 0.5mg | 500kg/tablet | 160mg | 8 sec. | 7 sec. | 0.12% |
| Lactose 60mg Crystalline cellulose 19.5mg Magnesium stearate 0.5mg | 500kg/tablet | 160mg | 13 sec. | 4 sec. | 0.00% |
| Sample's inner core composition: 72mg of erythritol, 7.5mg of corn starch, 0.5mg of magnesium stearate | | | | | |

### Test Example 6

### [Method]

The rate of 72 in 80 of erythritol (NIKKEN CHEMICALS), 7.5 in 80 of corn starch (NIHON SHOKUHIN KAKO) and 0.5 in 80 of magnesium stearate (TAIHEI CHEMICAL INDUSTRIAL) were mixed for use as bulk material for the inner core. Separately therefrom, the rate of 19.5 in 80 of crystalline cellulose (Asahi KASEI), 60 in 80 of erythritol and 0. 5 in 80 of magnesium stearate (TAIHEI CHEMICAL INDUSTRIAL) were mixed for use as bulk material for the outer layer. Next, 20mg of the bulk material for the outer layer was weighed into a space enclosed by lower center and outer punches, with the lower center punch among the pressing punches lowered, in an apparatus equipped with a die and upper and lower punches having a double structure - 6.0mm in inner diameter and 8.0mm in outer diameter - manipulatable for pressing operation and having flat edge angle. Then, by moving the upper and lower center punches in mutually approaching directions precompression was manually conducted to such an extent that the surface became flat. Next, 80mg of the bulk material for the inner core was weighed into a space enclosed by the lower outer punch and above the temporary molded product of the outer layer, and precompression was manually conducted in the same manner as above by moving the upper and lower center punches in mutually approaching directions. Further, the remaining 60mg of bulk material for the outer layer was weighed into a space enclosed by the inner wall of the die and above the temporary molded products of the outer layer and the inner core such that the temporary molded product of the inner core was completely covered by the bulk material for the outer layer. Then, by moving the upper and lower punches in mutually approaching directions, tabletting was conducted at a compression pressure of about 500 to 2000kg/tablet - this time using a hydraulic manual press (same as above). Molded products were similarly made at varying blending ratios of erythritol and crystalline cellulose in the outer layer as shown in Table 6.

The molded products made as samples were evaluated for dissolubility or/and disintegrability in the same manner as with Test Example 1. Moldability evaluation was conducted in the same manner as with Test Example 4. The results are shown in Table 6.

### [Results and Discussion]

The molded products were discovered to offer friability of 0.87% or less and a disintegration time of 32 seconds or less according to the Japanese Pharmacopoeia which was excellent both in moldability and dissolubility or/and disintegrability, as a result of selection of crystalline cellulose as ingredient rich in moldability and erythritol as ingredient rich in dissolubility or/and disintegrability and use of a 6:2 to 2:6 blending ratio of erythritol to crystalline cellulose. The most preferred erythritol-to-crystalline cellulose blending ratio would probably be 6:2 to 4:4.

**Table 6**

| Outer layer composition of samples | Tabletting pressure | Tablet weight | Disintegration time (Japanese Pharmacopoeia) | Disintegration time (oral cavity) | Friability (100 rev.) |
|---|---|---|---|---|---|
| Erythritol 60mg Crystalline cellulose 19.5mg Magnesium stearate 0.5mg | 2000kg/tablet | 160mg | 7 sec. | 10 sec. | 0.87% |
| Erythritol 40mg Crystalline cellulose 39.5mg Magnesium stearate 0.5mg | 500kg/tablet | 160mg | 11 sec. | 8 sec. | 0.62% |
| Erythritol 20mg Crystalline cellulose 59.5mg Magnesium stearate 0.5mg | 500kg/tablet | 160mg | 32 sec. | 36 sec. | 0.00% |
| Sample's inner core composition: 72mg of erythritol, 7.5mg of corn starch, 0.5mg of magnesium stearate | | | | | |

### Test Example 7

### [Method]

The rate of 72 in 80 of erythritol (NIKKEN CHEMICALS), 7.5 in 80 of corn starch (NIHON SHOKUHIN KAKO) and 0.5 in 80 of magnesium stearate (TAIHEI CHEMICAL INDUSTRIAL) were mixed for use as bulk material for the inner core. The rate of 19.5 in 80 of crystalline cellulose (Asahi KASEI), 60 in 80 of mannitol (TOWA CHEMICAL INDUSTRY) and 0.5 in 80 of magnesium stearate (TAIHEI CHEMICAL INDUSTRIAL) were mixed for use as bulk material for the outer layer. Next, 20mg of the bulk material for the outer layer was weighed into a space enclosed by lower center and outer punches, with the lower center punch among the pressing punches lowered, in an apparatus equipped with a die and upper and lower punches of 6.0mm in inner diameter and 8.0mm in outer diameter having a double structure, manipulatable for pressing operation and having flat edge angle. Then, by moving the upper and lower center punches in mutually approaching directions, temporary compression was manually conducted to such an extent that the surface became flat. Next, 80mg of the bulk material for the inner core was weighed into a space enclosed by the lower outer punch and above the temporary molded product of the outer layer, and temporary compression was manually conducted in the same manner as above by moving the upper and lower center punches in mutually approaching directions. Further, the remaining 60mg of bulk material for the outer layer was weighed into a space enclosed by the inner wall of the die and above the temporary molded products of the outer layer and the inner core such that the temporary molded product of the inner core was completely covered by the bulk material for the outer layer. Then, by moving the upper and lower punches in mutually approaching directions, tabletting was conducted at a compression pressure of about 500 to 1000kg/tablet - this time using a hydraulic manual press (same as above). Molded products were similarly made at varying blending ratios of mannitol and crystalline cellulose in the outer layer as shown in Table 7.

The molded products made as samples were evaluated for dissolubility or/and disintegrability in the same manner as with Test Example 1. Moldability evaluation was conducted in the same manner as with Test Example 4. The results are shown in Table 7.

### [Results and Discussion]

The molded products were discovered to offer friability of 0.31% or less and a disintegration time of 26 seconds or less according to the Japanese Pharmacopoeia ,which was excellent both in moldability and dissolubility or/and disintegrability, as a result of selection of crystalline cellulose as ingredient rich in moldability and mannitol as ingredient rich in dissolubility or/and disintegrability and use of a 6:2 to 2:6 blending ratio of mannitol to crystalline cellulose. The most preferred mannitol-to-crystalline cellulose blending ratio would probably be 6:2 to 4:4.

**Table 7**

| Outer layer composition of samples | Tabletting pressure | Tablet weight | Disintegration time (Japanese Pharmacopoeia) | Disintegration time (oral cavity) | Friability (100 rev.) |
|---|---|---|---|---|---|
| Mannitol 60mg Crystalline cellulose 19.5mg Magnesium stearate 0 .5mg | 1000kg/tablet | 160mg | 9 sec. | 8 sec. | 0.31% |
| Mannitol 50mg Crystalline cellulose 29.5mg Magnesium stearate 0.5mg | 500kg/tablet | 160mg | 8 sec. | 7 sec. | 0.12% |
| Mannitol 40mg Crystalline cellulose 39.5mg Magnesium stearate 0.5mg | 500kg/tablet | 160mg | 10 sec. | 10 sec. | 0.00% |
| Mannitol 20mg Crystalline cellulose 59.5mg Magnesium stearate 0.5mg | 500kg/tablet | 160mg | 26 sec. | 16 sec. | 0.00% |
| Sample's inner core composition: 72mg of erythritol, 7.5mg of corn starch, 0.5mg of magnesium stearate | | | | | |

### Test Example 8

### [Method]

The rate of 72 in 80 of erythritol (NIKKEN CHEMICALS), 7.5 in 80 of corn starch (NIHON SHOKUHIN KAKO) and 0.5 in 80 of magnesium stearate (TAIHEI CHEMICAL INDUSTRIAL) were mixed for use as bulk material for the inner core. The rate of 19.5 in 80 of crystalline cellulose (Asahi KASEI), 60 in 80 of lactose (DMV) and 0.5 in 80 of magnesium stearate (TAIHEI CHEMICAL INDUSTRIAL) were mixed for use as bulk material for the outer layer. Next, 20mg of the bulk material for the outer layer was weighed into a space enclosed by lower center and outer punches, with the lower center punch among the pressing punches lowered, in an apparatus equipped with a die and upper and lower punches of 6.0mm in inner diameter and 8.0mm in outer diameter having a double structure, manipulatable for pressing operation and having flat edge angle. Then, by moving the upper and lower center punches in mutually approaching directions, temporary compression was manually conducted to such an extent that the surface became flat. Next, 80mg of the bulk material for the inner core was weighed into a space enclosed by the lower outer punch and above the temporary molded product of the outer layer, and temporary compression was manually conducted in the same manner as above by moving the upper and lower center punches in mutually approaching directions. Further, the remaining 60mg of bulk material for the outer layer was weighed into a space enclosed by the inner wall of the die and above the temporary molded products of the outer layer and the inner core such that the temporary molded product of the inner core was completely covered by the bulk material for the outer layer. Then, by moving the upper and lower punches in mutually approaching directions, tabletting was conducted at a compression pressure of about 500 to 1500kg/tablet - this time using a hydraulic manual press (same as above). Molded products were similarly made at varying blending ratios of lactose and crystalline cellulose in the outer layer as shown in Table 8.

The molded products made as samples were evaluated for dissolubility or/and disintegrability in the same manner as with Test Example 1. Moldability evaluation was conducted in the same manner as with Test Example 4. The results are shown in Table 8.

### [Results and Discussion]

The molded products were discovered to offer friability of 0.68% or less and a disintegration time of 42 seconds or less according to the Japanese Pharmacopoeia, which was excellent both in moldability and dissolubility or/and disintegrability, as a result of selection of crystalline cellulose as ingredient rich in moldability and lactose as ingredient rich in dissolubility or/and disintegrability and use of a 7:1 to 2 : 6 blending ratio of lactose to crystalline cellulose. The most preferred lactose-to-crystalline cellulose blending ratio would probably be 6:2 to 4:4.

From the aforementioned results, 9:1 and 6:2 blending ratios were selected respectively of erythritol to corn starch as ingredients for the inner core and lactose to crystalline cellulose as ingredients for the outer layer for Test Example 9, and the effect was evaluated of addition of 20mg/tablet of famotidine as an example of main ingredient on the moldability and dissolubility or/and disintegrability.

**Table 8**

| Outer layer composition of samples | Tabletting pressure | Tablet weight | Disintegration time (Japanese Pharmacopoeia) | Disintegration time (oral cavity) | Friability (100 rev.) |
|---|---|---|---|---|---|
| Lactose 70mg Crystalline cellulose 9.5mg Magnesium stearate 0.5mg | 1500kg/tablet | 160mg | 42 sec. | 13 sec. | 0.68% |
| Lactose 60mg Crystalline cellulose 19.5mg Magnesium stearate 0 .5mg | 500kg/tablet | 160mg | 13 sec. | 4 sec. | 0.00% |
| Lactose 50mg Crystalline cellulose 29.5mg Magnesium stearate 0.5mg | 500kg/tablet | 160mg | 7 sec. | 9 sec. | 0.00% |
| Lactose 20mg Crystalline cellulose 59.5mg Magnesium stearate 0. 5mg | 300kg/tablet | 160mg | 18 sec. | 8 sec. | 0.00% |
| Sample's inner core composition: 72mg of erythritol, 7.5mg of corn starch, 0.5mg of magnesium stearate | | | | | |

### Test Example 9

### [Method]

The rate of 20 in 80 of famotidine (Quimica Sintetica S.A.), 53.5 in 80 of erythritol (NIKKEN CHEMICALS) , 6 in 80 of corn starch (NIHON SHOKUHIN KAKO) and 0.5 in 80 of magnesium stearate (TAIHEI CHEMICAL INDUSTRIAL) were mixed for use as bulk material for the inner core. The rate of 19.5 in 80 of crystalline cellulose (Asahi KASEI), 60 in 80 of lactose (DMV) and 0.5 in 80 of magnesium stearate (TAIHEI CHEMICAL INDUSTRIAL) were mixed for use as bulk material for the outer layer. Next, 20mg of the bulk material for the outer layer was weighed into a space enclosed by lower center and outer punches, with the lower center punch among the pressing punches lowered, in an apparatus equipped with a die and upper and lower punches of 6.0mm in inner diameter and 8.0mm in outer diameter having a double structure, manipulatable for pressing operation and having flat edge angle. Then, by moving the upper and lower center punches in mutually approaching directions temporary compression was manually conducted to such an extent that the surface became flat. Next, 80mg of the bulk material for the inner core was weighed into a space enclosed by the lower outer punch and above the temporary molded product of the outer layer, and temporary compression was manually conducted in the same manner as above by moving the upper and lower center punches in mutually approaching directions. Further, the remaining 60mg of bulk material for the outer layer was weighed into a space enclosed by the inner wall of the die and above the temporary molded products of the outer layer and the inner core such that the temporary molded product of the inner core was completely covered by the bulk material for the outer layer. Then, by moving the upper and lower punches in mutually approaching directions, tabletting was conducted at a compression pressure of about 500kg/tablet - this time using a hydraulic manual press (same as above).

The molded products made as samples were evaluated for dissolubility or/and disintegrability in the same manner as with Test Example 1. Moldability evaluation was conducted in the same manner as with Test Example 4. The results are shown in Table 9.

### [Results and Discussion]

When added with 20mg/tablet of famotidine as an example of main ingredient to the inner core, the molded product was discovered to offer friability of 0.13% and a disintegration time of seven seconds according to the Japanese Pharmacopoeia, which was excellent both in moldability and dissolubility or/and disintegrability, as a result of use of a 9:1 blending ratio of erythritol to corn starch and 6:2 blending ratio of lactose to crystalline cellulose.

**Table 9**

| Inner core composition of samples | Tabletting pressure | Tablet weight | Disintegration time (Japanese Pharmacopoeia) | Disintegration time (oral cavity) | Friability (100 rev.) |
|---|---|---|---|---|---|
| Famotidine 20mg Erythritol 53.5mg Corn starch 6mg Magnesium stearate 0.5mg | 500kg/tablet | 160mg | 7 sec. | 7 sec. | 0.13% |
| Sample's outer layer composition: 60mg of lactose, 19.5mg of crystalline cellulose, 0.5mg of magnesium stearate | | | | | |

### Comparative Test Example

### [Method]

"Gaster D tablet 20mg" (Yamanouchi Pharmaceutical) , a commercial orally fast-disintegrating tablet, was evaluated as follows. The tablet was evaluated for dissolubility or/and disintegrability in the same manner as with Test Example 1. Moldability evaluation was conducted in the same manner as with Test Example 4. The results are shown in Table 10.

### [Results and Discussion]

Gaster D tablet was discovered to be a fast-disintegrating molded product meeting general standards, offering friability of 0.40% and a disintegration time of 53 seconds according to the Japanese Pharmacopoeia.

On the other hand, the molded product of the present invention, that in Test Example 9 (degree of friability: 0.13%, disintegration time according to the Japanese Pharmacopoeia: seven seconds), was considerably excellent as compared with Gaster D tablet in terms of moldability and dissolubility or/and disintegrability alike.

**Table 10**

| Sample | Tablet weight | Disintegration time (Japanese Pharmacopoeia) | Disintegration time (oral cavity) | Friability (100 rev.) |
|---|---|---|---|---|
| Gaster D 20mg (Yamanouchi Pharmaceutical) | 180mg | 53 sec. | 23 sec. | 0.40% |

### INDUSTRIAL APPLICABILITY

A press-coated fast-dissolving/disintegrating molded product of the present invention is efficiently producible and more excellent in dissolubility or/and disintegrability and further offers sufficient moldability (tablet strength). Therefore, the present invention makes the press-coated fast-dissolving/disintegrating molded product industrially viable in various fields and is most suitable, notably in the field of drugs, for an orally fast-dissolving/disintegrating tablet.

## Claims

1. A press-coated fast-dissolving/disintegrating molded product comprising:
an inner core that contains an ingredient rich in dissolubility or/and disintegrability and whose disintegration time is 1 minute or less; and
an outer layer around the inner core that contains an ingredient rich in moldability and whose disintegration time is 1 minute or less, wherein
the disintegration time of the entire molded product is 1 minute or less.

2. The press-coated fast-dissolving/disintegrating molded product according to claim 1, wherein
the outer layer further contains an ingredient rich in dissolubility or/and disintegrability.

3. The press-coated fast-dissolving/disintegrating molded product according to claim 1, wherein
the inner core further contains a dissolution/disintegration accelerant.

4. The press-coated fast-dissolving/disintegrating molded product according to any one of claims 1 to 3, wherein
the friability (25 rpm, 4 min.) of the molded product is 5% or less.

5. The press-coated fast-dissolving/disintegrating molded product according to claim 1, wherein
the ingredient rich in moldability contained in the outer layer is one or more ingredient (s) selected from a group consisting of sorbitol, crystalline cellulose, hydroxypropylcellulose, and povidone.

6. The press-coated fast-dissolving/disintegrating molded product according to claim 2, wherein
the ingredient rich in dissolubility or/and disintegrability contained in the outer layer is saccharides or/and sugar alcohols.

7. The press-coated fast-dissolving/disintegrating molded product according to claim 6, wherein
the saccharides or/and sugar alcohols are one or more ingredient (s) selected from a group consisting of glucose, xylose, lactose, sucrose, maltose, xylitol, mannitol, multitol, erythritol, and lactitol.

8. The press-coated fast-dissolving/disintegrating molded product according to claim 2, wherein
the ingredient rich in dissolubility or/and disintegrability contained in the outer layer is lactose, mannitol or/and erythrytol, and wherein
the ingredient rich in moldability contained in the outer layer is crystalline cellulose.

9. The press-coated fast-dissolving/disintegrating molded product according to claim 1, wherein
the ingredient rich in dissolubility or/and disintegrability contained in the inner core is saccharides or/and sugar alcohols.

10. The press-coated fast-dissolving/disintegrating molded product according to claim 9, wherein
the saccharides or/and sugar alcohols are one or more ingredient(s) selected from a group consisting of glucose, xylose, lactose, sucrose, maltose, xylitol, mannitol, multitol, erythritol, and lactitol.

11. The press-coated fast-dissolving/disintegrating molded product according to claim 3, wherein
the dissolution/disintegration accelerant contained in the inner core is one or more ingredient (s) selected from a group consisting of carmellose, carmellose calcium, carmellose sodium, crosscarmellose sodium, low substituted hydroxypropylcellulose, carboxymethyl starch sodium, corn starch, potato starch, alpha starch, partial alpha starch, hydroxypropyl starch, crosspovidone, sodium laurylsulfate, polysorbate, polyoxypropylene-polyoxyethylene glycol, sorbitan monooleate, propylene glycol monostearate, and polyethylene glycol monolaurate.

12. The press-coated fast-dissolving/disintegrating molded product according to claim 3, wherein
the ingredient rich in dissolubility or/and disintegrability contained in the inner core is erythritol, and wherein
the dissolution/disintegration accelerant contained in the inner core is corn starch.

13. The press-coated fast-dissolving/disintegrating molded product according to any one of claims 1 to 3, wherein
the inner core and the outer layer are integrally molded.

14. The press-coated fast-dissolving/disintegrating molded product according to any one of claims 1 to 3, wherein
the outer layer is a compressed coating layer not containing any main ingredients, and wherein
the inner core contains the whole quantity of the main ingredient.

15. The press-coated fast-dissolving/disintegrating molded product according to any one of claims 1 to 3, wherein
the inner core consists of an imperfect molded substance.

16. The press-coated fast-dissolving/disintegrating molded product according to any one of claims 1 to 3, wherein
the outer perimeter layer entirely has a thickness of 1mm or less.

17. The press-coated fast-dissolving/disintegrating molded product according to any one of claims 1 to 3, wherein
fast dissolubility/disintegrability is exhibited in the oral cavity.
